# EUROPEAN PATENT SPECIFICATION

(11) **EP 4 561 997 B1**
(45) Date of publication and mention of the grant of the patent: **02.09.2026**
(21) Application number: 23750941.9
(22) Date of filing: 26.07.2023
(51) Int. Cl.: C07D 311/74, C07D 311/94, A61K 8/49, A61Q 13/00, C11B 9/00

(54) **CHROMEN-5-ONE DERIVATIVES FOR USE AS FRAGRANCES**
CHROMEN-5-ON-DERIVATE ZUR VERWENDUNG ALS DUFTSTOFFE
DÉRIVÉS DE CHROMÈNE-5-ONE UTILISÉS COMME PARFUMS

(30) Priority: 29.07.2022 WO PCT/CN2022/108926
(43) Date of publication of application: 04.06.2025
(73) Proprietor: Givaudan SA, 1214 Vernier (CH)
(72) Inventor: ZHOU, Lijun, Shanghai 201203 (CN); CHAI, An, Shanghai 201203 (CN); YU, Zhiyu, Shanghai 201203 (CN)
(74) Representative: Global Patents
(86) International application number: PCT/EP2023/070739
(87) International publication number: WO 2024/023169

(56) References cited:
- WO-A1-2014/064716
- WO-A1-2022/024054
- US-A- 5 030 618

## Description

### TECHNICAL FIELD

The present invention is concerned with fragrance ingredients, and to compositions containing them that are useful in fragrance applications. It furthermore relates to a method of making them. In particular, the present invention is concerned with substituted tetrahydro-(5H)-chromen-5-one and hexahydro-(5H)-chromen-5-one derivatives of formula (I) possessing woody, peppery notes which are reminiscent of Rotundone, an oxygenated sesquiterpene also known as (3S,5R,8S)-3,8-dimethyl-5-(prop-1-en-2-yl)-3,4,5,6,7,8-hexahydroazulen-1(2H)-one. This invention relates furthermore to a method of their production and fragrance compositions comprising them.

### BACKGROUND

In the flavor and fragrance industry, perfumers and flavorists are continually looking for new compounds possessing unique olfactory properties. Rotundone can be found in traces, for example, in pepper, marjoram, oregano, rosemary, and patchouli oil, but also in aromas of grapefruit, orange, apple and mango. Sensory analyses showed that Rotundone, when added at even subthreshold levels it has significant effects on the overall odor impression. Accordingly, there is a need for ingredients that are suitable for the flavor and fragrance industry possessing woody, peppery notes which are reminiscent of Rotundone.

Surprisingly inventors found a new class of ingredients of formula (I) as defined herein below possessing the highly sought-after woody, peppery notes reminiscent of Rotundone.

### SUMMARY

In accordance with a first aspect of the present invention there is provided a compound of formula (I) 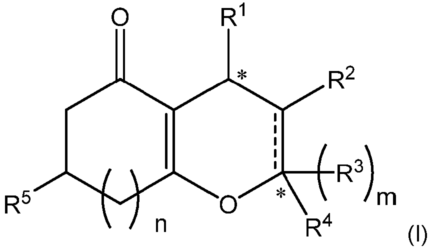 wherein
R¹ is selected from C₁ - C₅ alkyl (e.g. C₁, C₂, C₃ alkyl),
R² is selected from hydrogen, C₁-C₃ alkyl (e.g. H or methyl),
R³ is selected from hydrogen, methyl and ethyl,
R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-2-en-1-yl),
R⁵ is selected from hydrogen and methyl,
n is 0 or 1, and
the dotted line represents together with the C-C bond a single bond and m is 1, or
the dotted line represents together with the C-C bond a double bond and m is 0.

In accordance with a second aspect of the present invention there is provided a fragranced article comprising as odorant a compound of formula (I) of the first aspect.

In accordance with a third aspect of the present invention there is provided a method of improving, enhancing and/or modifying a consumer product base or consumable product base by means of adding thereto an olfactory acceptable amount of a compound for formula (I) of the first aspect, or a mixture thereof.

In accordance with a fourth aspect of the present invention there is provided a fragrance or flavor composition comprising a compound of formula (I) of the first aspect, or a mixture thereof.

Certain embodiments of any aspect of the present invention may provide one or more of the following advantages:
- provision of novel fragrance compounds with woody, peppery notes which is reminiscent of Rotundone,
- provision of a highly performing compound possessing sort of transparency.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that substituted tetrahydro-(5H)-chromen-5-one and hexahydro-(5H)-chromen-5-one derivatives as defined herein possessing woody, peppery notes which are reminiscent of Rotundone.

In accordance with a first aspect of the present invention there is provided a compound of formula (I) 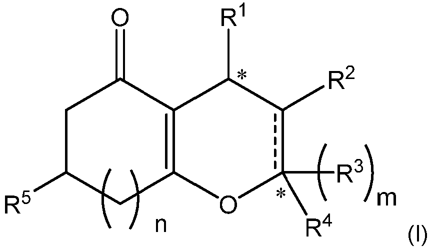 wherein
R¹ is selected from C₁ - C₅ alkyl (e.g. C₁, C₂, C₃ alkyl),
R² is selected from hydrogen, C₁-C₃ alkyl (e.g. H or methyl),
R³ is selected from hydrogen, methyl and ethyl,
R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-2-en-1-yl),
R⁵ is selected from hydrogen and methyl,
n is 0 or 1, and
the dotted line represents together with the C-C bond a single bond and m is 1, or
the dotted line represents together with the C-C bond a double bond and m is 0.

In one particular embodiment there is provide a compound of formula (I) wherein R¹ is selected from C₁ to C₅ alkyl (e.g., C₁, C₂, C₃ alkyl), R², R³ and R⁵ are hydrogen, R⁴ is selected from C₁ to C₆ alkyl, C₂ to C₆ alkenyl, and C₃ to C₆ cycloalkyl, n is 0 or 1, and the dotted line represents together with the C-C bond a single bond and m is 1, or the dotted line represents together with the C-C bond a double bond and m is 0.

In a further embodiment there is provided a compound of formula (I) wherein R¹ is selected from C₁ to C₅ alkyl (e.g., C₁, C₂, C₃ alkyl), R², R³ and R⁵ are hydrogen, R⁴ is selected from C₁ to C₆ alkyl, C₂ to C₆ alkenyl, and cyclopropyl, n is 0 or 1, and the dotted line represents together with the C-C bond a single bond and m is 1, or the dotted line represents together with the C-C bond a double bond and m is 0.

In a further embodiment there is provided a compound of formula (I) wherein R¹ is selected from C₁ to C₅ alkyl (e.g. C₁, C₂, C₃ alkyl), R², R³ and R⁵ are hydrogen, R⁴ is selected from C₁ to C₆ alkyl, C₂ to C₆ alkenyl, and cyclopropyl, n is 1, the dotted line represents together with the C-C bond a single bond and m is 1, or the dotted line represents together with the C-C bond a double bond and m is 0.

In a further embodiment there is provided a compound of formula (I) wherein R¹ is selected from C₁ to C₅ alkyl (e.g. C₁, C₂, C₃ alkyl), R², R³ and R⁵ are hydrogen, R⁴ is selected from C₁ to C₆ alkyl, C₂ to C₆ alkenyl, and cyclopropyl, n is 1, and the dotted line represents together with the C-C bond a single bond and m is 1.

In a further embodiment there is provided a compound of formula (I) wherein R¹ is selected from C₁ to C₅ alkyl (e.g. C₁, C₂, C₃ alkyl), R², R³ and R⁵ are hydrogen, R⁴ is selected from C₁ to C₆ alkyl, C₂ to C₆ alkenyl, n is 1, and the dotted line represents together with the C-C bond a single bond and m is 1.

In a further embodiment there is provide a compound of formula (II) 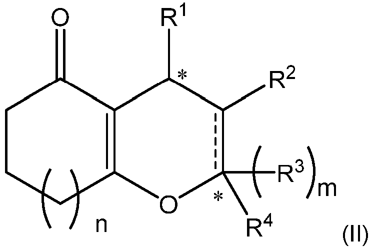 wherein
R¹ is selected from C₁ - C₅ alkyl (e.g. C₁, C₂, C₃ alkyl),
R² is selected from hydrogen, C₁-C₃ alkyl (e.g. H or methyl),
R³ is selected from hydrogen, methyl and ethyl,
R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-2-en-1-yl),
R⁵ is selected from hydrogen and methyl,
n is 0 or 1, and
the dotted line represents together with the C-C bond a single bond and m is 1, or
the dotted line represents together with the C-C bond a double bond and m is 0.

In a further embodiment there is provided a compound of formula (III) 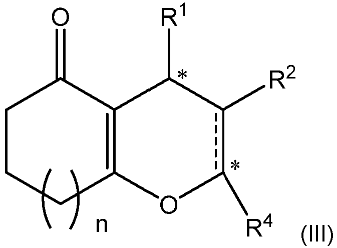 wherein
R¹ is selected from C₁ - C₅ alkyl (e.g. C₁, C₂, C₃ alkyl),
R² is selected from hydrogen, C₁-C₃ alkyl (e.g. H or methyl),
R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-2-en-1-yl),
n is 0 or 1 (preferably n is 1), and
the dotted line represents together with the C-C bond a single bond or a double bond, preferably a single bond.

In a further embodiment there is provided a compound of formula (III) wherein R¹ is selected from C₁ to C₅ alkyl (e.g. C₁, C₂, C₃ alkyl), R² is hydrogen, C₁-C₃ alkyl (e.g. H or methyl), R⁴ is selected from C₁ - C₆ alkyl, C₂-C₆ alkenyl, and C₃-C₆ cycloalkyl (e.g.cyclopropyl), n is 0 or 1 (preferably n is 1), and
the dotted line represents together with the C-C bond a single bond or a double bond, preferably a single bond.

As one particular example of a compound of formula (I) one may cite, as non-limiting example, 2-ethyl-4-methyl-2,3,4,6,7,8-hexahydro-5H-chromen-5-one, which possess a peppery, woody, green odor profile.

As a further specific example of a compound of formula (I) one may cite, as non-limiting example, 4-methyl-2-propyl-2,3,4,6,7,8-hexahydro-5H-chromen-5-one, which possess a woody, peppery, fiber wood, cedarwood odor profile.

As a further specific example of a compound of formula (I) one may cite, as non-limiting example, 2,4-diethyl-2,3,4,6,7,8-hexahydro-5H-chromen-5-one, which possess a woody, cedarwood, green, bell pepper, vetiver odor profile reminiscent of Rotundone.

Apart from these woody, cedarwood, green, bell pepper, vetiver odor characteristic, the compound is quite interesting since it possesses excellent trail properties.

By "trail" it is meant, that a fragrance ingredient, when placed in a directional air flow, may be received at a distance from its source with a reliable and recognizable odor. These properties are of interest, in particular in the fields of personal and fine fragrances. The ability of a fragrance ingredient or perfume to be perceived in a space some distance from its source is an important characteristic of a perfume.

In another particular embodiment the compound of formula (I) is selected from the group consisting of 2-(but-2-en-2-yl)-4-ethyl-2,3,4,6,7,8-hexahydro-5H-chromen-5-one; 2-(but-2-en-2-yl)-4-methyl-2,3,4,6,7,8-hexahydro-5H-chromen-5-one; 2-(but-3-en-2-yl)-4-ethyl-2,3,4,6,7,8-hexahydro-5H-chromen-5-one; 2-(but-3-en-2-yl)-4-methyl-2,3,4,6,7,8-hexahydro-5H-chromen-5-one; 2-(sec-butyl)-4-ethyl-2,3,4,6,7,8-hexahydro-5H-chromen-5-one; 2-(sec-butyl)-4-methyl-2,3,4,6,7,8-hexahydro-5H-chromen-5-one; 2-(tert-butyl)-4-ethyl-2,3,4,6,7,8-hexahydro-5H-chromen-5-one; 2,2,4-trimethyl-2,3,4,6,7,8-hexahydro-5H-chromen-5-one; 2,3,4-trimethyl-4,6,7,8-tetrahydro-5H-chromen-5-one; 2,4-diethyl-3,4,6,7-tetrahydrocyclopenta[b]pyran-5(2H)-one; 2,4-diethyl-3-methyl-2,3,4,6,7,8-hexahydro-5H-chromen-5-one; 2,4-diethyl-4,6,7,8-tetrahydro-5H-chromen-5-one; 2,4-dimethyl-2,3,4,6,7,8-hexahydro-5H-chromen-5-one; 2,4-dimethyl-2,3,4,6,7,8-hexahydro-5H-chromen-5-one; 2,4-dimethyl-4,6,7,8-tetrahydro-5H-chromen-5-one; 2-allyl-4-ethyl-2,3,4,6,7,8-hexahydro-5H-chromen-5-one; 2-allyl-4-methyl-2,3,4,6,7,8-hexahydro-5H-chromen-5-one; 2-butyl-4-ethyl-2,3,4,6,7,8-hexahydro-5H-chromen-5-one; 2-butyl-4-methyl-2,3,4,6,7,8-hexahydro-5H-chromen-5-one; 2-cyclopropyl-4-ethyl-2,3,4,6,7,8-hexahydro-5H-chromen-5-one; 2-cyclopropyl-4-methyl-2,3,4,6,7,8-hexahydro-5H-chromen-5-one; 2-ethyl-3,4-dimethyl-2,3,4,6,7,8-hexahydro-5H-chromen-5-one; 2-ethyl-4-(prop-1-en-1-yl)-2,3,4,6,7,8-hexahydro-5H-chromen-5-one; 2-ethyl-4,7-dimethyl-2,3,4,6,7,8-hexahydro-5H-chromen-5-one; 2-ethyl-4-propyl-2,3,4,6,7,8-hexahydro-5H-chromen-5-one; 2-isobutyl-4-methyl-2,3,4,6,7,8-hexahydro-5H-chromen-5-one; 2-isopropyl-4-methyl-2,3,4,6,7,8-hexahydro-5H-chromen-5-one; 2-isopropyl-4-methyl-2,3,4,6,7,8-hexahydro-5H-chromen-5-one; 4-ethyl-2-(2-methylallyl)-2,3,4,6,7,8-hexahydro-5H-chromen-5-one; 4-ethyl-2-(2-methylprop-1-en-1-yl)-2,3,4,6,7,8-hexahydro-5H-chromen-5-one; 4-ethyl-2-(prop-1-en-1-yl)-2,3,4,6,7,8-hexahydro-5H-chromen-5-one; 4-ethyl-2-(prop-1-en-2-yl)-2,3,4,6,7,8-hexahydro-5H-chromen-5-one; 4-ethyl-2,3-dimethyl-2,3,4,6,7,8-hexahydro-5H-chromen-5-one; 4-ethyl-2-isobutyl-2,3,4,6,7,8-hexahydro-5H-chromen-5-one; 4-ethyl-2-isopropyl-2,3,4,6,7,8-hexahydro-5H-chromen-5-one; 4-ethyl-2-methyl-2,3,4,6,7,8-hexahydro-5H-chromen-5-one; 4-ethyl-2-propyl-2,3,4,6,7,8-hexahydro-5H-chromen-5-one; 4-ethyl-2-propyl-3,4,6,7-tetrahydrocyclopenta[b]pyran-5(2H)-one; 4-ethyl-2-vinyl-2,3,4,6,7,8-hexahydro-5H-chromen-5-one; 4-isopropyl-2-methyl-2,3,4,6,7,8-hexahydro-5H-chromen-5-one; 4-methyl-2-(2-methylprop-1-en-1-yl)-2,3,4,6,7,8-hexahydro-5H-chromen-5-one; and 4-methyl-2-(prop-1-en-2-yl)-2,3,4,6,7,8-hexahydro-5H-chromen-5-one, and isomers thereof.

The compounds as defined by formula (I) (which encompasses compounds of formula (II) and compounds of formula (III)) comprise several chiral centers (two of which are indicated by * in the respective formulae) and as such may exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or stereoselective synthesis (including a catalytic reaction).

It was observed that some of the individual isomers have different odor qualities, both in terms of performance as well as olfactory properties. Odour detection thresholds measurements of have revealed that compounds of formula (I) wherein the substituents R¹ and R⁴ are in *trans*-position possessing an odour detection threshold which is up to 30 times lower compared to the same compound wherein the substituents R¹ and R⁴ are in *cis-*position.

Thus there is provided in a further embodiment of the present invention a compound of formula (I') 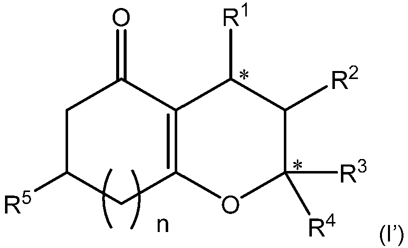 wherein
R¹ is selected from C₁ - C₅ alkyl (e.g. C₁, C₂, C₃ alkyl),
R² is selected from hydrogen, C₁-C₃ alkyl (e.g. H or methyl),
R³ is selected from hydrogen, methyl and ethyl,
R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-3-en-1-yl),
R⁵ is selected from hydrogen and methyl,
n is 0 or 1, and
R¹ and R⁴ are in *trans*-position to each other.

In one particular embodiment there is provided a compound of formula (I') wherein R¹ is selected from C₁ - C₅ alkyl (e.g. C₁, C₂, C₃ alkyl), R² is selected from hydrogen and C₁-C₃ alkyl (e.g. H or methyl), R³ is hydrogen, R⁴ is selected from C₁ - C₆ alkyl (e.g. C₁, C₂, C₃, or C₄ alkyl), C₂-C₆ alkenyl e.g. C₂, C₃, or C₄ alkenyl), and cyclopropyl, R⁵ is selected from hydrogen and methyl, n is 0 or 1, and R¹ and R⁴ are in *trans*-position to each other.

The compounds of formula (I) (which encompasses compounds of formula (II) and (III)) may be used alone, as isomeric mixture thereof, or in combination with known odorant molecules selected from the extensive range of natural products, and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art.

As used herein, "carrier material" means a material which is practically neutral from a odorant point of view, i.e. a material that does not significantly alter the organoleptic properties of odorants.

The term "auxiliary agent" refers to ingredients that might be employed in a fragrance composition for reasons not specifically related to the olfactive performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a fragrance composition. A detailed description of the nature and type of adjuvants commonly used in fragrance compositions containing same cannot be exhaustive, but it has to be mentioned that said ingredients are well known to a person skilled in the art.

As used herein, 'fragrance composition' means any composition comprising a compound of formula (I) (which encompasses compounds of formula (II) and (III)), or a mixture thereof and a base material, e.g. a diluent conventionally used in conjunction with odorants, such as diethyl phthalate (DEP), dipropylene glycol (DPG), isopropyl myristate (IPM), pentane-1,2-diol, triethyl citrate (TEC) and alcohol (e.g. ethanol). Optionally, the composition may comprise an anti-oxidant adjuvant. Said anti-oxidant may be selected from Tinogard^{®} TT (BASF), Tinogard^{®} Q (BASF), Tocopherol (including its isomers, CAS 59-02-9; 364-49-8; 18920-62-2; 121854-78-2), 2,6-bis(1,1-dimethylethyl)-4-methylphenol (BHT, CAS 128-37-0) and related phenols, hydroquinones (CAS 121-31-9).

The following list comprises examples of known odorant molecules, which may be combined with a compound of formula (I), or a mixture thereof:
- essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, *gurjun* balsam oil, lavender oil and/ or ylang-ylang oil;
- alcohols, e.g. cinnamic alcohol ((E)-3-phenylprop-2-en-1-ol); cis-3-hexenol ((Z)-hex-3-en-1-ol); citronellol (3,7-dimethyloct-6-en-1-ol); dihydro myrcenol (2,6-dimethyloct-7-en-2-ol); EbanolTM ((E)-3-methyl-5-(2,2,3-trimethylcyclopent-3-en-1-yl)pent-4-en-2-ol); eugenol (4-allyl-2-methoxyphenol); ethyl linalool ((E)-3,7-dimethylnona-1,6-dien-3-ol); farnesol ((2E,6Z)-3,7,11-trimethyldodeca-2,6,10-trien-1-ol); geraniol ((E)-3,7-dimethylocta-2,6-dien-1-ol); Super MuguetTM ((E)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); RhodinolTM (3,7-dimethyloct-6-en-1-ol); SandaloreTM (3-methyl-5-(2,2,3-trimethylcyclopent-3-en-1-yl)pentan-2-ol); terpineol (2-(4-methylcyclohex-3-en-1-yl)propan-2-ol); or TimberolTM (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;
- aldehydes and ketones, e.g. anisaldehyde (4-methoxybenzaldehyde); alpha amyl cinnamic aldehyde (2-benzylideneheptanal); GeorgywoodTM (1-(1,2,8,8-tetramethyl-1,2,3,4,5,6,7,8-octahydronaphthalen-2-yl)ethanone); Hydroxycitronellal (7-hydroxy-3,7-dimethyloctanal); Iso E Super^{®} (1-(2,3,8,8-tetramethyl-1,2,3,4,5,6,7,8-octahydronaphthalen-2-yl)ethanone); Isoraldeine^{®} ((E)-3-methyl-4-(2,6,6-trimethylcyclohex-2-en-1-yl)but-3-en-2-one); HedioneTM (methyl 3-oxo-2-pentylcyclopentaneacetate); 3-(4-isobutyl-2-methylphenyl)propanal; maltol; methyl cedryl ketone; methylionone; verbenone; and/or vanillin;
- ether and acetals, e.g. Ambrox^{®} (3a,6,6,9a-tetramethyl-2,4,5,5a,7,8,9,9b-octahydro-1H-benzo[e][1]benzofuran); geranyl methyl ether ((2E)-1-methoxy-3,7-dimethylocta-2,6-diene); and/ or Spirambrene^{®} (2',2',3,7,7-pentamethylspiro[bicyclo[4.1.0]heptane-2,5'-[1,3]dioxane]) ;
- esters and lactones, e.g. benzyl acetate; cedryl acetate ((1S,6R,8aR)-1,4,4,6-tetramethyloctahydro-1H-5,8a-methanoazulen-6-yl acetate); delta-decalactone (6-pentyltetrahydro-2H-pyran-2-one); Helvetolide^{®} (2-(1-(3,3-dimethylcyclohexyl)ethoxy)-2-methylpropyl propionate); delta-undecalactone (5-heptyloxolan-2-one); and / or vetiveryl acetate ((4,8-dimethyl-2-propan-2-ylidene-3,3a,4,5,6,8a-hexahydro-1H-azulen-6-yl) acetate);
- macrocycles, e.g. Ambrettolide ((Z)-oxacycloheptadec-10-en-2-one); ethylene brassylate (1,4-dioxacycloheptadecane-5,17-dione); and / or Exaltolide^{®} (16-oxacyclohexadecan-1-one); and
- heterocycles, e.g. isobutylquinoline (2-isobutylquinoline).

Thus there is provided in a further aspect of the invention a fragrance composition comprising a compound of formula (I) (which encompasses compounds of formula (II), and (III)).

The compounds of formula (I) (which encompasses compounds of formula (II), and (III)) may be used in a broad range of fragranced articles, e.g. in any field of fine and functional perfumery, such as perfumes, air care products, household products, laundry products, body care products and cosmetics. The compound can be employed in widely varying amounts, depending upon the specific article and on the nature and quantity of other odorant ingredients. The proportion is typically from 0.00001 to 30 weight % of the article. In one embodiment, the compound may be employed in a fabric softener in an amount from 0.0001 to 1 weight per cent (e.g. 0.001 to 0.1 including 0.05 and 0.03 weight %). In another embodiment, the compound may be employed in a hair care product ((dry) shampoo, hair conditioner) in an amount from about 0.1 to about 5 weight %. In another embodiment, the compound may be used in fine perfumery in amounts from 0.00001 to 30 weight per cent (e.g. up to about 10 or up to 20 weight per cent), more preferably between 0.3 and 5 weight per cent (e.g. 0.5 to 3 weight per cent). However, these values are given only by way of example, since the experienced perfumer may also achieve effects or may create novel accords with lower or higher concentrations.

The compounds of formula (I) (which encompasses compounds of formula (II), and (III)) may be employed in a consumer product base simply by directly mixing the compound, or a fragrance composition comprising a compound of formula (I) (which encompasses compounds of formula (II), and (III)), or a mixture thereof, with the consumer product base, or it may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, and then mixed with the consumer product base.

Thus, the invention additionally provides a method of manufacturing a fragranced article, comprising the incorporation a compound of formula (I) (which encompasses compounds of formula (II), and (III)), or a mixture thereof as a fragrance ingredient, either by directly admixing to the consumer product base or by admixing a fragrance composition comprising a compound of formula (I) (which encompasses compounds of formula (II), and (III)), or a mixture thereof, which may then be mixed with a consumer product base, using conventional techniques and methods. Through the addition of an olfactory acceptable amount of a compound of formula (I) (which encompasses compounds of formula (II), and (III)), or a mixture thereof the odor notes of a consumer product base will be improved, enhanced, or modified.

Thus, the invention furthermore provides a method for improving, enhancing or modifying a consumer product base by means of the addition thereto of an olfactorily acceptable amount of a compound of formula (I) (which encompasses compounds of formula (II), and (III)), or a mixture thereof.

There is provided in a further aspect of the present invention a fragranced article comprising:
a) a compound of formula (I), or a mixture thereof 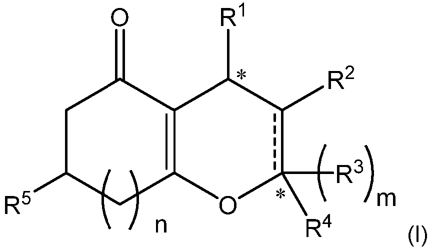 wherein
   R¹ is selected from C₁ - C₅ alkyl (e.g. C₁, C₂, C₃ alkyl),
   R² is selected from hydrogen, C₁-C₃ alkyl (e.g. H or methyl),
   R³ is selected from hydrogen, methyl and ethyl,
   R⁴ is selected from C₁ - C₆ alkyl, C₂-C₆ alkenyl, C₃-C₆ cycloalkyl (e.g.cyclopropyl), and C₅-C₆ cycloalkenyl (e.g. cyclopent-2-en-1-yl),
   R⁵ is selected from hydrogen and methyl,
   n is 0 or 1, and
   the dotted line represents together with the C-C bond a single bond and m is 1, or
   the dotted line represents together with the C-C bond a double bond and m is 0. and
b) a consumer product base.

As used herein, 'consumer product base' means a composition for use as a consumer product to fulfill specific actions, such as cleaning, softening, and caring or the like. Examples of such products include fine perfumery, e.g. perfume and eau de toilette; fabric care, household products and personal care products such as cosmetics, laundry care detergents, rinse conditioner, personal cleansing composition, detergent for dishwasher, surface cleaner; laundry products, e.g. softener, bleach, detergent; body-care products, e.g. shampoo, shower gel; air care products (includes products that contain preferably volatile and usually pleasant-smelling compounds which advantageously can even in very small amounts mask unpleasant odors). Air fresheners for living areas contain, in particular, natural and synthetic essential oils such as pine needle oils, citrus oil, eucalyptus oil, lavender oil, and the like, in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants.

Cosmetic products include:
(a) cosmetic skincare products, especially bath products, skin washing and cleansing products, skincare products, eye makeup, lip care products, nail care products, intimate care products, foot care products;
(b) cosmetic products with specific effects, especially sunscreens, tanning products, de-pigmenting products, deodorants, antiperspirants, hair removers, and shaving products;
(c) cosmetic dental-care products, especially dental and oral care products, tooth care products, cleaners for dental prostheses, adhesives for dental prostheses; and
(d) cosmetic hair care products, especially hair shampoos, hair care products, hair setting products, hair-shaping products, and hair coloring products.

This list of products is given by way of illustration, and is not to be regarded as being in any way limiting.

In one particular embodiment the consumer product base is selected form fine perfumery, and personal care products, including deodorants, hair care products, soaps, and the like.

In a further particular embodiment the consumer product base is selected from fabric care products, including fabric softener, and home care products, including air fresheners, dish washers and the like.

In another particular embodiment the fragrance composition comprises a mixture of compounds of formula (I) as herein above defined. For example, the fragrance composition may comprise a mixture comprising 2-ethyl-4-methyl-2,3,4,6,7,8-hexahydro-5H-chromen-5-one and at least a second compound of formula (I), for example, 4-methyl-2-propyl-2,3,4,6,7,8-hexahydro-5H-chromen-5-one, 2-isopropyl-4-methyl-2,3,4,6,7,8-hexahydro-5H-chromen-5-one, and/or 2,4-diethyl-2,3,4,6,7,8-hexahydro-5H-chromen-5-one.

The compounds of formula (I) (which encompasses compounds of formula (II) and (III)) wherein the dotted line represents together with the C-C bond a single bond may, for example, be synthesized by Grignard reaction of 2-hydroxy hexahydro-5H-chromen-5-one or 2-hydroxy tetrahydrocyclopenta[b]pyran-5(2H)-one followed by treatment of acid to form hexahydro-(5*H*)-chromen-5-one or tetrahydrocyclopenta[*b*]pyran-5(*4H*)-one derivatives. The hemiacetal (2-hydroxy hexahydro-5H-chromen-5-one or 2-hydroxy tetrahydro cyclopenta[b]pyran-5(2H)-one) may, for example, be synthesized by reaction of 1,3-cyclic dione with α,β-unstaturated aldehyde catalyzed by amino acid and it's derivative (eg. 2-(diphenyl((trimethylsilyl)oxy)methyl)pyrrolidine), or may be synthesized by domino reaction of 1,3-cyclic diketone, aldehyde, and enol ether with or without catalyst (eg. proline).

Alternatively, the compounds of formula (I) (which encompasses compounds of formula (II) and (III)) wherein the dotted line represents together with the C-C bond a double bond may, for example, be synthesized by reaction of 1,3-cyclic dione with α,β-unstaturated ketone catalyzed by Lewis acid (eg. ZnCl₂) to form substituted tetrahydro-(5*H*)-chromen-5-one or substituted dihydrocyclopenta[b]pyran-5(*4H*)-one.

The pure cis isomers of formula (I') may, for example, be synthesised by hydrogenation of corresponding tetrahydro-(5*H*)-chromen-5-ones or dihydrocyclopenta[*b*]pyran-5(*4H*)-ones with a catalyst (eg, 10% Pd/C).

Further particulars as to reaction conditions are provided in the examples.

The invention is now further described with reference to the following non-limiting examples. These examples are for the purpose of illustration only and it is understood that variations and modifications can be made by one skilled in the art.

### General Method A:

All reactions were performed under argon using solvents and reagents from commercial suppliers without further purification. Solvents for extraction and chromatography were technical grade and used without further purification. Flash chromatography was performed using Tsingdao Haiyang Chemical silica gel (200 - 300 mesh) and Santai Technologies silica flash columns. Unless otherwise noted, a mixture of Heptane : MTBE was used as eluent. NMR spectra were recorded with AW400 MHz Bruker spectrometer instrument. The chemical shifts for ¹H NMR spectra was reported in δ (ppm) referenced to the residual proton signal of the deuterated solvent; coupling constants were expressed in Hertz (Hz). ¹³C NMR spectra were referenced to the carbon signals of the deuterated solvent. The following abbreviations are used: s = singlet, d = doublet, t = triplet, q = quartet, m = multiplet, dd = double doublet, bs = broad singlet. GC/MS spectral data were obtained from an Agilent 6890 N and MSD 5975 using a column HP-5 MS, 30 m, 0.25 mm, 0.25 µm.

### Example 1-1: 2-ethyl-4-methyl-2,3,4,6,7,8-hexahydro-5H-chromen-5-one

1. To a solution of cyclohexane-1,3-dione (100 g, 0.89 mol, 1.0 equiv) and but-2-enal (69 g, 0.98 mol, 1.1 equiv) in Dichloromethane (250 mL) was added 2-(diphenyl((trimethylsilyl)oxy)methyl)pyrrolidine (8.7 g, 27mmol, 0.03 equiv) in Dichloromethane (100 ml) slowly at 0°C and stirred at r.t. under argon for 16 h. The solvent was removed and the residue was purified by silica gel column chromatography (Heptane:MTBE=5:1 to 1:1) to give 2-hydroxy-4-methyl-2,3,4,6,7,8-hexahydro-5H-chromen-5-one (146 g, 0.80 mol, 90 % yield, mixture of diasteromers,dr=4:1) as a white solid.
   ¹H NMR (400 MHz, CDCl₃, mixture of diasteromers) δ 5.54 - 5.02 (m, 2H), 2.98 - 2.64 (m, 1H), 2.60 - 2.15 (m, 4H), 2.14 - 1.61 (m, 4H), 1.35 - 0.99 (m, 3H) ppm. ¹³C NMR (101 MHz, CDCl₃, mixture of diasteromers) δ 198.9, 198.7, 170.2, 169.4, 116.5, 115.9, 94.5, 93.2, 37.1, 36.9, 35.7, 35.6, 28.9, 28.7, 23.2, 23.0, 21.0, 20.9, 20.4, 20.3 ppm.
2. To a solution of 2-hydroxy-4-methyl-2,3,4,6,7,8-hexahydro-5H-chromen-5-one (120 g, 0.66 mol) in THF (1L) was added ethylmagnesium chloride (0.88 L, 1.32 mol, 1.5 M) at 0°C and stirred at rt. for 16 h under argon. Aqueous hydrochloric acid was added at 10~15 °C to quench the reaction until pH=3. The mixture was stirred at r.t. for 16h, and then extracted with ethyl acetate (0.5 L*3), the organic layer was washed with saturated aqueous NaHCO₃, brine and dried over MgSO₄. After filtration, the solvent was removed and the crude oil was purified via distillation to give 2-ethyl-4-methyl-2,3,4,6,7,8-hexahydro-5H-chromen-5-one (115 g, 0.59 mol, yield: 90%, mixture of diasteromers, trans:cis=1.75:1) as a colorless liquid.
   ¹H NMR (400 MHz, CDCl₃, mixture of diasteromers) δ 4.02 - 3.56 (m, 1H), 2.85 - 2.57 (m, 1H), 2.44 - 1.22 (m, 10H), 1.22 - 0.88 (m, 6H) ppm. ¹³C NMR (101 MHz, CDCl₃, mixture of diasteromers) δ 198.0, 171.9, 171.2, 117.3, 116.2, 78.5, 74.3, 37.7, 37.4, 37.2, 33.7, 29.0, 28.9, 28.2, 27.9, 25.8, 22.6, 21.1, 21.0, 20.4, 20.1, 9.5 ppm. GC/MS (EI): m/z (%): 194 (98) [M⁺], 179 (80), 165 (20), 151 (90), 139 (100), 125 (15), 55 (26).

### Odor description: peppery, woody, green.

The cis/trans isomers in 2-ethyl-4-methyl-2,3,4,6,7,8-hexahydro-5H-chromen-5-one (example 1-1) was further separated via silica gel chromatography (Heptane: MTBE=20:1).

### Trans-2-ethyl-4-methyl-2,3,4,6,7,8-hexahydro-5H-chromen-5-one: colorless liquid.

¹H NMR (400 MHz, CDCl₃) δ 3.99 - 3.89 (m, 1H), 2.84 - 2.73 (m, 1H), 2.43 - 2.31 (m, 4H), 1.99 - 1.87 (m, 2H), 1.79 - 1.48 (m, 4H), 1.08 (d, *J* = 6.9 Hz, 3H), 1.01 (t, *J* = 7.4 Hz, 3H) ppm. ¹³C NMR (101 MHz, CDCl₃) δ 198.0, 171.2, 116.2, 74.3, 37.2, 33.6, 28.8, 28.2, 22.6, 21.1, 21.0, 9.4 ppm. GC/MS (EI): m/z (%): 194 (96) [*M*⁺], 179 (94), 165 (24), 151 (100), 139 (75), 55 (33).

### Odor description: woody, green.

### Cis-2-ethyl-4-methyl-2,3,4,6,7,8-hexahydro-5H-chromen-5-one: colorless liquid.

¹H NMR (400 MHz, CDCl₃) δ 3.70 - 3.56 (m, 1H), 2.62 - 2.49 (m, 1H), 2.37 - 1.17 (m, 10 H), 1.10 (d, *J* = 6.5 Hz, 3H), 0.92 (t, *J* = 7.4 Hz, 3H) ppm. ¹³C NMR (101 MHz, CDCl3) δ = 197.9, 171.9, 117.2, 78.4, 37.6, 37.3, 28.9, 27.8, 25.7, 20.3, 20.0, 9.4 ppm. GC/MS (EI): m/z (%): 194 (65) [*M*⁺], 179 (42), 166 (12), 151 (60), 139 (100), 55 (23).

### Odor description: green, damp, woody

### Example 1-2: 4-methyl-2-propyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 19mmol) and propylmagnesium bromide (19 mL (2M), 38mmol, ) according to the procedure of example 1-1 as colorless liquid (15 mmol, 3.1 g, 77% yield, trans:cis=2.1:1).

¹H NMR (400 MHz, CDCl₃, mixture of diasteromers) δ 4.09 - 3.71 (m, 1H), 2.85 - 2.56 (m, 1H), 2.48 - 1.25 (m, 12H), 1.22 - 1.06 (m, 3H), 1.05-0.88 (m, 3H) ppm.¹³C NMR (101 MHz, CDCl₃, mixture of diasteromers)δ 198.0, 172.0, 171.2, 117.3, 116.1, 77.1, 72.9, 38.2, 37.3, 37.2, 37.1, 34.1, 29.0, 28.8, 25.8, 22.6, 21.1, 21.0, 20.3, 20.1, 18.4, 18.3, 14.0, 14.0 ppm. GC/MS (EI): m/z (%): 208 (82) [*M*⁺], 193 (56), 179 (27), 165 (17), 151 (86), 139 (100), 55 (30).

### Odor description: woody, peppery, fiber wood, cedarwood

*Trans- and Cis-* 4-methyl-2-propyl-2,3,4,6,7,8-hexahydro-5H-chromen-5-one was isolated from 4-methyl-2-propyl-2,3,4,6,7,8-hexahydro-5H-chromen-5-one via silica gel chromatography (Heptane: MTBE=20:1).

### Trans-4-methyl-2-propyl-2,3,4,6,7,8-hexahydro-5H-chromen-5-one: colorless liquid.

¹H NMR (400 MHz, CDCl₃) δ 4.18 - 3.76 (m, 1H), 2.97 - 2.60 (m, 1H), 2.49 - 2.19 (m, 4H), 2.10 - 1.85 (m, 2H), 1.77 - 1.26 (m, 6H), 1.19 - 1.04 (m, 3H), 1.02 - 0.82 (m, 3H) ppm.¹³C NMR (101 MHz, CDCl₃) δ 198.0, 171.2, 116.2, 72.9, 37.4, 37.2, 34.2, 28.9, 22.6, 21.1, 21.0, 18.4, 14.0 ppm. GC/MS (EI): m/z (%): 208 (94) [*M*⁺], 193 (68), 179 (34), 165 (22), 151 (99), 139 (100), 125 (31), 55 (35).

### Odor description: woody(cedrenol), peppery

### Cis-4-methyl-2-propyl-2,3,4,6,7,8-hexahydro-5H-chromen-5-one: colorless liquid.

¹H NMR (400 MHz, CDCl₃) δ 3.76 - 3.63 (m, 1H), 2.63 - 2.50 (m, 1H), 2.41 - 2.08 (m, 4H), 2.02 - 1.69 (m, 3H), 1.67 - 1.16 (m, 5H), 1.10 (d, *J* = 6.5 Hz, 3H), 0.89 - 0.86 (m, 3H), ppm.¹³C NMR (101 MHz, CDCl₃) δ = 197.9, 171.9, 117.3, 77.1, 38.2, 37.4, 37.1, 29.0, 25.8, 20.4, 20.1, 18.3, 14.0 ppm. GC/MS (EI): m/z (%): 208 (55) [*M*⁺], 193 (32), 179 (13), 165 (12), 139 (100), 55 (25).

### Odor description: green, woody, cedrenol, vetiver, grapefruit

### Example 1-3: 2-isopropyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (21g, 115mmol) and isopropylmagnesium bromide (77 mL, 230mmol, 3.0 M) according to the procedure of example 1-1 as colorless liquid (75 mmol, 16 g, 65% yield).

¹H NMR (400 MHz, CDCl₃, mixture of diasteromers) *δ* 3.76 - 3.38 (m, 1H), 2.79 - 2.46 (m, 1H), 2.39 - 2.08 (m, 4H), 1.99 - 1.16 (m, 5H), 1.15 - 0.97 (m, 3H), 0.95 - 0.77 (m, 6H) ppm. ¹³C NMR (101 MHz, CDCl₃, mixture of diasteromers) δ 197.7, 172.1, 171.2, 117.2, 116.0, 81.8, 77.5, 37.3, 37.1, 34.8, 31.9, 31.8, 30.5, 28.8, 28.6, 26.0, 22.4, 21.0, 20.9, 20.3, 20.0, 18.0, 17.9, 17.7, 17.7 ppm. GC/MS (EI): m/z (%): 208 (63) [*M*+], 193 (27), 165 (21), 152 (29), 139 (100), 55 (20).

### Odor description: peppery, woody, violet, vetiver, fruity

Trans-2-isopropyl-4-methyl-2,3,4,6,7,8-hexahydro-5H-chromen-5-one was isolated from 4-methyl-2-propyl-2,3,4,6,7,8-hexahydro-5H-chromen-5-one via silica gel chromatography (Heptane: MTBE=20:1) as colorless liquid.

¹H NMR (400 MHz, CDCl₃) δ 3.93 - 3.46 (m, 1H), 2.80 - 2.63 (m, 1H), 2.34 - 2.18 (m, 4H), 1.94 - 1.70 (m, 3H), 1.57 - 1.40 (m, 2H), 1.00 (d, *J* = 6.9 Hz, 3H), 0.95 - 0.82 (m, 6H) ppm. ¹³C NMR (101 MHz, CDCl₃)δ 197.9, 171.3, 116.1, 77.6, 37.1, 32.0, 30.6, 28.7, 22.4, 21.0, 20.9, 18.1, 17.8 ppm. GC/MS (EI): m/z (%): 208 (71) [*M*+], 193 (37), 179 (2), 165 (27), 152 (36), 139 (100), 55 (23).

### Odor description: fruity woody ambery, peppery

### Example 1-4: 2-(sec-butyl)-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 19 mmol) and sec-butylmagnesium bromide (38 mL, 38 mmol, 1.0 M) according to the procedure of example 1-1 as colorless liquid (15mmol, 3.4 g, 80% yield, trans:cis=3:1).

¹H NMR (400 MHz, CDCl₃, mixture of isomers) δ 3.88 - 3.50 (m, 1H), 2.79 - 2.48 (m, 1H), 2.38 - 1.13 (m, 11H), 1.12 - 0.98 (m, 3H), 0.92 - 0.79 (m, 6H) ppm. ¹³C NMR (101 MHz, CDCl₃, mixture of isomers)δ 198.1, 172.5, 172.3, 171.6, 171.5, 117.4, 116.2, 116.1, 80.8, 80.6, 76.5, 76.3, 38.5, 38.4, 38.4, 38.3, 37.3, 37.2, 35.2, 34.2, 30.7, 29.8, 28.9, 28.8, 28.7, 26.2, 26.1, 25.2, 25.1, 25.0, 22.6, 22.4, 21.1, 21.0, 20.9, 20.4, 20.4, 20.1, 14.3, 14.2, 14.1, 14.0, 11.7, 11.6, 11.5, 11.4 ppm. GC/MS (EI): m/z (%): 222 (37) [M⁺], 207 (11), 193 (11), 179 (2), 165 (15), 151 (23), 139 (100), 55 (22).

### Odor description: vetiver, woody, fruity isoraldéïne, powdery orris, peppery, green

### Example 1-5: 2-isobutyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 19 mmol) and isobutylmagnesium bromide (48 ml, 38 mmol, 0.8 M) according to the procedure of example 1-1 as colorless liquid (17mmol, 3.8 g, 89% yield, trans:cis=2.5:1).

¹H NMR (400 MHz, CDCl₃, mixture of diasteromers) *δ* 4.12 - 3.67 (m, 1H), 2.79 - 2.45 (m, 1H), 2.38 - 2.14 (m, 4H), 2.01 - 1.40 (m, 6H), 1.33 - 1.17 (m, 1H), 1.13 - 0.98 (m, 3H), 0.92 - 0.82 (m, 6H) ppm. ¹³C NMR (101 MHz, CDCl₃, mixture of diasteromers) δ 198.1, 172.0, 171.2, 117.3, 116.1, 75.6, 71.5, 44.3, 44.1, 38.7, 37.3, 37.2, 34.6, 29.0, 28.9, 25.7, 24.3, 24.3, 23.0, 23.0, 22.6, 22.4, 22.3, 21.1, 21.0, 20.4, 20.1 ppm. GC/MS (EI): m/z (%): 222 (64) [*M*⁺], 207 (22), 179 (48), 166 (46), 151 (73), 139 (100), 109 (20), 55 (23).

### Odor description: green, woody, ambery, peppery

### Example 1-6: 4-methyl-2-(prop-1-en-2-yl)-2,3,4,6,7,8-hexahydro-5H-chromen-5-one

The compound was obtained from 2-hydroxy-4-methyl-2,3,4,6,7,8-hexahydro-5H-chromen-5-one (10g, 38mmol) and prop-1-en-2-ylmagnesium bromide (154 ml, 77mmol, 0.5 M) according to the procedure of example 1-1 as colorless liquid (22 mmol, 4.5 g, 57% yield, trans:cis=2:1).

¹H NMR (400 MHz, CDCl₃, mixture of diasteromers) δ 5.03 - 4.84 (m, 2H), 4.39 - 4.09 (m, 1H), 2.81 - 2.55 (m, 1H), 2.38 -1.38 (m, 11H), 1.14 - 1.01 (m, 3H) ppm.¹³C NMR (101 MHz, CDCl₃, mixture of diasteromers) δ 197.8, 171.5, 170.8, 143.5, 143.2, 117.2, 116.1, 112.7, 112.5, 79.9, 76.0, 37.2, 37.1, 36.8, 33.0, 28.9, 28.7, 25.82, 22.6, 20.9, 20.8, 20.1, 18.2, 18.0 ppm. GC/MS (EI): m/z (%): 206 (65) [*M*⁺], 178 (8), 163 (38), 151 (100), 135 (20), 79 (14), 68 (76).

### Odor description: green, metallic, rooty, woody, orris isoraldéïne, peppery

### Example 1-7: 2,4-dimethyl-2,3,4,6,7,8-hexahydro-5H-chromen-5-one

The compound was obtained from 2-hydroxy-4-methyl-2,3,4,6,7,8-hexahydro-5H-chromen-5-one (10.0 g, 54.9 mmol) and methylmagnesium bromide (37 mL, 110mmol, 3.0 M) according to the procedure of example 1-1 as colorless liquid (16 mmol, 2.8 g, 28% yield, trans:cis=1:2.7).

¹H NMR (400 MHz, CDCl₃, mixture of diasteromers) δ 4.16 - 3.75 (m, 1H), 2.74 - 2.50 (m, 1H), 2.37 - 1.22 (m, 11H), 1.12 - 0.98 (m, 3H) ppm.¹³C NMR (101 MHz, CDCl₃, mixture of diasteromers) δ = 197.9, 171.8, 171.0, 117.1, 115.9, 73.5, 69.4, 40.0, 37.3, 37.1, 35.9, 28.9, 28.8, 25.7, 22.7, 21.1, 21.0, 20.8, 20.3, 20.0 ppm. GC/MS (EI): m/z (%): 180 (38) [*M*⁺], 165 (62), 151 (100), 137 (42), 124 (35), 109 (46), 79 (63), 67 (81).

### Odor description: woody, cedarwood, green

### Example 1-8: 2-cyclopropyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 27.4 mmol) and cyclopropylmagnesium bromide (110 mL, 55mmol, 0.5 M) according to the procedure of example 1-1 as colorless liquid (14mmol, 2.9 g, 51% yield, trans:cis=3:2).

¹H NMR (400 MHz, CDCl₃, mixture of diasteromers) δ 3.32 - 3.02 (m, 1H), 2.79 - 2.48 (m, 1H), 2.42 - 1.34 (m, 8H), 1.17 - 0.90 (m, 4H), 0.64 - 0.16 (m, 4H) ppm.¹³C NMR (101 MHz, CDCl₃, mixture of diasteromers) δ = 197.8, 171.6, 170.9, 117.1, 116.0, 81.6, 77.7, 37.9, 37.2, 37.0, 34.0, 28.9, 28.7, 25.7, 22.5, 20.9, 20.8, 20.2, 20.0, 15.0, 14.8, 3.2, 3.2, 1.7, 1.6 ppm. GC/MS (EI): m/z (%): 206 (20) [*M*⁺], 191 (16), 163 (17), 151 (14), 139 (59), 121 (18), 107 (9), 91 (17), 79 (28), 67 (100), 53 (53).

### Odor description: peppery, woody, green

### Example 1-9: 2-allyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 27mmol) and allylmagnesium bromide (55 mL, 55mmol, 1.0 M) according to the procedure of example 1-1 as colorless liquid (7.7 mmol, 1.6 g, 28% yield, trans:cis=1.5:1).

¹H NMR (400 MHz, CDCl₃, mixture of diasteromers) δ 5.96 - 5.69 (m, 1H), 5.27 - 5.04 (m, 2H), 4.15 - 3.79 (m, 1H), 2.84 - 2.56 (m, 1H), 2.51 - 1.26 (m, 10H), 1.21 - 1.05 (m, 3H) ppm. ¹³C NMR (101 MHz, CDCl₃, mixture of diasteromers) δ = 196.9, 170.7, 169.9, 132.4, 132.2, 116.9, 116.3, 115.2, 75.5, 71.5, 38.5, 38.3, 36.5, 36.3, 36.1, 32.5, 27.9, 27.7, 24.6, 21.5, 20.1, 20.0, 19.3, 19.0 ppm. GC/MS (EI): m/z (%): 206 (38) [*M*⁺], 191 (12), 163 (100), 149 (17), 139 (66), 125 (23), 109 (24), 91 (18), 79 (33), 67 (27), 55 (26).

### Odor description: green, vetiver, cedarwood

### Example 1-10: 2-butyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 27mmol) and butylmagnesium bromide (69 mL, 55 mmol, 0.8 M) according to the procedure of example 1-1 as colorless liquid (16mmol, 3.7 g, 60% yield, trans:cis=2.4:1).

¹H NMR (400 MHz, CDCl₃, mixture of diasteromers) δ 3.99 - 3.63 (m, 1H), 2.78 - 2.49 (m, 1H), 2.36 - 1.22 (m, 14H), 1.13 - 0.98 (m, 3H), 0.90 - 0.81 (m, 3H) ppm. ¹³C NMR (101 MHz, CDCl₃, mixture of diasteromers) δ 198.0, 172.0, 171.2, 117.3, 116.1, 77.3, 73.2, 38.2, 37.3, 37.2, 34.9, 34.7, 34.1, 29.0, 28.8, 27.3, 27.2, 25.8, 22.6, 22.5, 21.1, 21.0, 20.3, 20.1, 14.0, 13.9 ppm. GC/MS (EI): m/z (%): 222 (73) [*M*⁺], 207 (45), 193 (7), 179 (23), 165 (14), 151 (68), 139 (100), 109 (28), 55 (20).

### Odor description: green, cork, myrrh, woody, peppery

### Example 1-11: 2-(but-2-en-2-yl)-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 25 mmol) and but-2-en-2-ylmagnesium bromide (99 mL, 50 mmol, 0.5 M) according to the procedure of example 1-1 as colorless liquid (14 mmol, 3.0 g, 55% yield).

¹H NMR (400 MHz, CDCl₃, mixture of isomers) δ 5.75 - 5.17 (m, 1H), 4.96 - 4.15 (m, 1H), 2.84 - 2.57 (m, 1H), 2.43 - 2.17 (m, 4H), 1.95 - 1.74 (m, 3H), 1.69 - 1.31 (m, 7H), 1.18 - 0.97 (m, 3H) ppm. ¹³C NMR (101 MHz, CDCl₃, mixture of isomers) δ 197.8, 172.2, 171.2, 134.0, 133.8, 123.5, 123.1, 123.0, 117.3, 116.0, 78.3, 75.2, 71.0, 37.3, 37.1, 36.4, 32.8, 32.0, 29.0, 28.8, 26.1, 22.6, 21.0, 20.9, 20.2, 20.0, 18.1, 18.0, 13.2, 12.9, 12.8, 11.5 ppm. GC/MS (EI): m/z (%): 220 (41) [*M*⁺], 205 (10), 191 (4), 177 (6), 165 (14), 151 (100), 82 (76), 67 (86).

### Odor description: woody, peppery, green, violet

### Example 1-12: 4-methyl-2-(2-methylprop-1-en-1-yl)-2,3,4,6,7,8-hexahydro-5H-chromen-5-one

The compound was obtained from 2-hydroxy-4-methyl-2,3,4,6,7,8-hexahydro-5H-chromen-5-one (5.0 g, 19 mmol) and (2-methylprop-1-en-1-yl)magnesium bromide (77 mL, 38mmol, 0.5 M) according to the procedure of example 1-1 as colorless liquid (10mmol, 2.3 g, 54% yield, trans:cis=3.5:1).

¹H NMR (400 MHz, CDCl₃, mixture of diasteromers) δ 5.31 - 4.96 (m, 1H), 4.76 - 4.26 (m, 1H), 2.78 - 2.56 (m, 1H), 2.40 - 1.36 (m, 14H), 1.15 - 0.96 (m, 3H) ppm.¹³C NMR (101 MHz, CDCl₃, mixture of diasteromers) δ 197.7, 171.7, 171.0, 137.7, 123.6, 123.4, 117.1, 115.9, 74.3, 70.2, 38.6, 37.2, 37.1, 34.6, 29.0, 28.8, 25.7, 25.5, 22.6, 21.0, 20.9, 20.2, 20.1, 18.2, 18.1 ppm. GC/MS (EI): m/z (%): 220 (43) [*M*⁺], 205 (4), 177 (7), 151 (95), 82 (100), 67 (61).

### Odor description: green, vetiver, grapefruit, woody, peppery

### Example 1-14: 2-(but-3-en-2-yl)-4-methyl-2,3,4,6,7,8-hexahydro-5H-chromen-5-one

The compound was obtained from 2-hydroxy-4-methyl-2,3,4,6,7,8-hexahydro-5H-chromen-5-one (5.0 g, 27mmol) and but-3-en-2-ylmagnesium bromide (61 mL, 55 mmol, 0.9 M) according to the procedure of example 1-1 as colorless liquid (19 mmol, 4.1 g, 68% yield).

¹H NMR (400 MHz, CDCl₃, mixture of isomers) δ 6.03 - 5.49 (m, 1H), 5.19 - 4.86 (m, 2H), 4.07 - 3.32 (m, 1H), 2.84 - 2.45 (m, 1H), 2.43 - 1.16 (m, 9H), 1.15 - 0.87 (m, 6H) ppm. ¹³C NMR (101 MHz, CDCl₃, mixture of isomers) δ = 197.9, 172.0, 171.9, 171.2, 171.1, 139.4, 139.3, 139.0, 117.4, 117.3, 116.2, 116.1, 115.6, 115.6, 115.5, 115.4, 80.3, 76.1, 42.0, 41.9, 41.6, 41.4, 37.3, 37.1, 35.3, 35.0, 31.2, 30.7, 28.8, 28.7, 28.6, 25.9, 22.4, 22.4, 21.0, 20.9, 20.2, 20.0, 15.5, 15.4, 15.3 ppm. GC/MS (EI): *m*/*z* (%): 220 (40) [*M*⁺], 205 (11), 191 (7), 163 (100), 139 (84), 109 (28), 93 (27), 79 (28), 67 (36), 55 (43).

### Odor description: violet, woody, cedarwood, peppery, green

### Example 1-15: 4-ethyl-2-methyl-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 4-ethyl-2-hydroxy-2,3,4,6,7,8-hexahydro-5H-chromen-5-one was obtained from reaction of cyclohexane-1,3-dione (100 g, 0.89 mol, 1.0 equiv) and pent-2-enal (83 g, 0.98 mol, 1.1 equiv) as white solid (0.67 mol, 132 g, 75 % yield).
   4-ethyl-2-hydroxy-2,3,4,6,7,8-hexahydro-5H-chromen-5-one:¹H NMR (400 MHz, CDCl₃, mixture of diasteromers) δ 5.55 - 5.26 (m, 1H), 4.78 (brs, 1H), 2.74 - 2.54 (m, 1H), 2.50 - 1.06 (m, 10H), 1.02 - 0.65 (m, 3H) ppm. ¹³C NMR (101 MHz, CDCl₃, mixture of diasteromers) δ 198.8, 198.6, 170.3, 170.1, 115.9, 115.3, 94.9, 93.3, 37.1, 37.0, 31.7, 31.3, 29.7, 29.3, 28.9, 28.8, 27.0, 25.7, 20.9, 20.3, 11.6, 11.1 ppm. GC/MS (EI): m/z (%): 196 (44) [*M*⁺], 178 (8), 167 (92), 153 (68), 139 (100), 125 (33), 79 (30), 55 (51).
2. The compound was obtained from 4-ethyl-2-hydroxy-2,3,4,6,7,8-hexahydro-5H-chromen-5-one (30g, 153mmol) and methylmagnesium bromide (306 mL, 306mmol, 1.0 M) according to the procedure of example 1-1 as colorless liquid (98mmol, 19g, 64% yield, trans:cis=1.8:1).

¹H NMR (400 MHz, CDCl₃, mixture of diasteromers) δ 4.30 - 3.69 (m, 1H), 2.68 - 2.41 (m, 1H), 2.40 - 2.08 (m, 4H), 2.06 - 1.53 (m, 4H), 1.45 - 0.98 (m, 5H), 0.94 - 0.54 (m, 3H) ppm. ¹³C NMR (101 MHz, CDCl₃, mixture of diasteromers) δ = 198.0, 197.9, 172.5, 170.9, 116.0, 115.4, 73.5, 69.6, 37.3, 37.2, 36.3, 31.5, 31.4, 29.4, 29.1, 28.9, 27.0, 26.0, 21.2, 21.0, 20.9, 20.0, 11.7, 10.3 ppm. GC/MS (EI): m/z (%): 194 (32) [*M*⁺], 179 (9), 165 (100), 153 (6), 137 (25), 123 (7), 109 (11), 91 (10), 79 (9), 67 (7), 55 (12).

### Odor description: peppery, woody, cedarwood

*Trans- and Cis-* 4-ethyl-2-methyl-2,3,4,6,7,8-hexahydro-5H-chromen-5-one was isolated from 4-ethyl-2-methyl-2,3,4,6,7,8-hexahydro-5H-chromen-5-one via silica gel chromatography (Heptane: MTBE=20:1) .

*Trans*-4-ethyl-2-methyl-2,3,4,6,7,8-hexahydro-5H-chromen-5-one: ¹H NMR (400 MHz, CDCl₃) δ 4.26 - 3.97 (m, 1H), 2.59 - 2.44 (m, 1H), 2.42 - 2.23 (m, 4H), 2.03 - 1.80 (m, 3H), 1.75 - 1.57 (m, 1H), 1.50 - 1.23 (m, 4H), 1.21 - 1.01 (m, 1H), 0.92 (t, *J* = 7.4 Hz, 3H) ppm. ¹³C NMR (101 MHz, CDCl₃) δ 197.8, 170.9, 115.5, 69.6, 37.3, 31.5, 29.5, 28.9, 27.1, 21.2, 21.1, 11.7 ppm. GC/MS (EI): m/z (%): 194 (33) [*M*⁺], 179 (9), 165 (100), 139 (18), 109 (10), 55 (10).

### Odor description: peppery, woody, cedarwood, earthy

*Cis*-4-ethyl-2-methyl-2,3,4,6,7,8-hexahydro-5H-chromen-5-one: ¹H NMR (400 MHz, CDCl₃) δ 3.95 - 3.74 (m, 1H), 2.60 - 2.41 (m, 1H), 2.36 - 2.12 (m, 4H), 2.06 - 1.61 (m, 4H), 1.38 - 1.12 (m, 5H), 0.85 - 0.67 (m, 3H) ppm. ¹³C NMR (101 MHz, CDCl₃) δ 197.9, 172.5, 115.9, 73.5, 37.3, 36.3, 31.3, 29.0, 26.0, 20.8, 20.0, 10.3 ppm. GC/MS (EI): m/z (%): 194 (34) [*M*⁺], 179 (9), 165 (100), 153 (8), 137 (26), 109 (11), 55 (13).

### Odor description: woody, cedarwood, dusty, green

### Example 1-16: 2,4-diethyl-2,3,4,6,7,8-hexahydro-5H-chromen-5-one

The compound was obtained from 4-ethyl-2-hydroxy-2,3,4,6,7,8-hexahydro-5H-chromen-5-one (25g, 127mmol) and ethylmagnesium chloride (127 mL, 255 mmol,) according to the procedure of example 1-1 as colorless liquid (102mmol, 21g, 80% yield, trans:cis=2:1).

¹H NMR (400 MHz, CDCl₃, mixture of diasteromers) δ 3.98 - 3.56 (m, 1H), 2.61 - 2.47 (m, 1H), 2.45 - 2.17 (m, 4H), 2.11 - 1.06 (m, 8H), 1.04 - 0.95 (m, 3H), 0.93 - 0.77 (m, 3H) ppm. ¹³C NMR (101 MHz, CDCl₃, mixture of diasteromers) δ 197.7, 197.7, 172.6, 171.0, 115.9, 115.5, 78.4, 74.3, 37.3, 37.1, 33.9, 31.3, 29.2, 28.9, 28.8, 28.1, 27.9, 26.9, 25.9, 20.9, 19.9, 11.6, 10.3, 9.4, 9.4 ppm. GC/MS (EI): m/z (%): 208 (40) [*M*⁺], 193 (11), 179 (100), 165 (7), 153 (17), 137 (21), 125 (22), 55 (14).

### Odor description: woody, cedarwood, green, bell pepper, vetiver

*Trans- and Cis-* 2,4-diethyl-2,3,4,6,7,8-hexahydro-5H-chromen-5-one was isolated from 2,4-diethyl-2,3,4,6,7,8-hexahydro-5H-chromen-5-one via silica gel chromatography (Heptane: MTBE=20:1).

*Trans*-2,4-diethyl-2,3,4,6,7,8-hexahydro-5H-chromen-5-one: ¹H NMR (400 MHz, CDCl₃) δ 4.03 - 3.70 (m, 1H), 2.62 - 2.47 (m, 1H), 2.46 - 2.21 (m, 4H), 2.02 - 1.58 (m, 6H), 1.47 - 1.31 (m, 1H), 1.17 - 1.05 (m, 1H), 1.01 (t, *J* = 7.4 Hz, 3H), 0.91 (t, *J* = 7.3 Hz, 3H) ppm. ¹³C NMR (101 MHz, CDCl₃) δ 197.8, 171.1, 115.7, 74.5, 37.3, 29.3, 29.1, 28.9, 28.3, 27.1, 21.1, 11.7, 9.5 ppm. GC/MS (EI): m/z (%): 208 (34) [*M*⁺], 193 (10), 179 (100), 165 (6), 151 (11), 137 (20), 125 (23), 55 (15).

### Odor description: woody, tobacco, straw, kephalis

*Cis*-2,4-diethyl-2,3,4,6,7,8-hexahydro-5H-chromen-5-one :¹H NMR (400 MHz, CDCl₃, mixture of diasteromers) δ 3.72 - 3.63 (m, 1H), 2.62 - 2.48 (m, 1H), 2.44 - 2.21 (m, 4H), 2.09 - 1.53 (m, 6H), 1.41 - 1.24 (m, 2H), 1.00 (t, *J* = 7.5 Hz, 3H), 0.82 (t, *J =* 7.4 Hz, 3H) ppm. ¹³C NMR (101 MHz, CDCl₃, mixture of diasteromers) δ = 197.8, 172.6, 116.0, 78.4, 37.3, 33.9, 31.4, 29.0, 28.0, 26.0, 20.0, 10.3, 9.4. GC/MS (EI): m/z (%): 208 (43) [*M*⁺], 193 (8), 179 (100), 165 (9), 153 (49), 137 (28), 125 (29), 55 (21).

### Odor description: fruity, berry, isoraldéïne, woody, cedarwood

### Example 1-17: 4-ethyl-2-vinyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol,) and vinylmagnesium bromide (41 mL, 41 mmol, 1.0 M) according to the procedure of example 1-1 as colorless liquid (18 mmol, 3.6 g, 86% yield, trans:cis=1.4:1).

¹H NMR (400 MHz, CDCl₃, mixture of diasteromers) δ 5.94 - 5.75 (m, 1H), 5.37 - 5.08 (m, 2H), 4.44 - 4.16 (m, 1H), 2.61- 1.01 (m, 11H), 0.89 - 0.73 (m, 3H) ppm. ¹³C NMR (101 MHz, CDCl₃, mixture of diasteromers) δ 197.9, 197.8, 171.9, 170.5, 136.9, 136.7, 116.8, 116.6, 116.2, 115.6, 77.4, 73.7, 63.6, 37.3, 37.2, 34.2, 31.0, 29.9, 29.1, 29.0, 28.8, 27.0, 25.9, 21.0, 20.9, 20.1, 11.7, 11.6, 10.4 ppm. GC/MS (EI): m/z (%): 206 (58) [*M*⁺], 191 (3), 177 (100), 165 (27), 149 (11), 137 (43), 121 (11), 107 (11), 91 (20), 77 (22), 67 (9), 55 (22).

### Odor description: woody, orris isoraldéïne, green cedarwood, peppery

### Example 1-18: 4-ethyl-2-propyl-2,3,4,6,7,8-hexahydro-5H-chromen-5-one

The compound was obtained from 4-ethyl-2-hydroxy-2,3,4,6,7,8-hexahydro-5H-chromen-5-one (10g, 51mmol) and propylmagnesium bromide (51mL, 102mmol, 2.0 M) according to the procedure of example 1-1 as colorless liquid (36 mmol, 7.9 g, 70% yield, trans:cis=2:1).

¹H NMR (400 MHz, CDCl₃, mixture of diasteromers) δ 4.08 - 3.63 (m, 1H), 2.63 - 2.47 (m, 1H), 2.47 - 2.15 (m, 4H), 2.10 - 1.61 (m, 5H), 1.60 - 1.20 (m, 5H), 1.04 - 0.78 (m, 6H) ppm.¹³C NMR (101 MHz, CDCl₃, mixture of diasteromers) δ 197.9, 197.8, 172.6, 171.0, 116.1, 115.6, 77.1, 73.1, 37.4, 37.3, 37.2, 34.5, 31.4, 29.6, 29.3, 29.1, 28.9, 27.0, 26.0, 21.0, 20.0, 18.4, 18.3, 14.0, 11.7, 10.3 ppm. GC/MS (EI): m/z (%): 222 (34) [*M*⁺], 207 (8), 193 (100), 179 (5), 165 (13), 153 (25), 137 (33), 125 (54), 55 (39).

### Odor description: woody, cedarwood, slightly green, grapefruit, vetiver, peppery

The tans/cis isomers were isolated from 4-ethyl-2-propyl-2,3,4,6,7,8-hexahydro-5H-chromen-5-one via silica gel chromatography (Heptane: MTBE=20:1).

### Trans-4-ethyl-2-propyl-2,3,4,6,7,8-hexahydro-5H-chromen-5-one

¹H NMR (400 MHz, CDCl3) δ 4.09 - 3.84 (m, 1H), 2.59 - 2.47 (m, 1H), 2.44 - 2.25 (m, 4H), 1.96 - 1.90 (m, 2H), 1.87 - 1.79 (m, 1H), 1.77 - 1.60 (m, 2H), 1.59 - 1.33 (m, 4H), 1.17 - 1.02 (m, 1H), 0.98 - 0.89 (m, 6H). ¹³C NMR (101 MHz, CDCl₃) δ 197.8, 171.0, 115.7, 73.1, 37.4, 37.2, 29.6, 29.3, 28.9, 27.0, 21.0, 18.4, 14.0, 11.7 ppm. GC/MS (EI): m/z (%): 222 (35) [*M*⁺], 207 (7), 193 (100), 137 (15), 125 (39), 55 (14).

### Cis-4-ethyl-2-propyl-2,3,4,6,7,8-hexahydro-5H-chromen-5-one

¹H NMR (400 MHz, CDCl3) δ 3.83 - 3.66 (m, 1H), 2.63 - 2.49 (m, 1H), 2.46 - 2.15 (m, 4H), 2.10 - 1.76 (m, 4H), 1.74 - 1.61 (m, 1H), 1.59 - 1.20 (m, 5H), 1.00 - 0.90(m, 3H), 0.82 (t, *J* = 7.5 Hz, 3H) ppm. ¹³C NMR (101 MHz, CDCl3) δ 197.8, 172.6, 116.1, 77.0, 37.4, 37.2, 34.5, 31.4, 29.0, 26.0, 20.0, 18.3, 13.9, 10.3 ppm. GC/MS (EI): m/z (%): 222 (46) [*M*⁺], 207 (9), 193 (100), 165 (14), 153 (60), 137 (29), 125 (48), 55 (21).

### Example 1-19: 4-ethyl-2-isopropyl-2,3,4,6,7,8-hexahydro-5H-chromen-5-one

The compound was obtained from 4-ethyl-2-hydroxy-2,3,4,6,7,8-hexahydro-5H-chromen-5-one (10g, 51mmol) and isopropylmagnesium bromide (102 mL, 102 mmol, 1.0 M) according to the procedure of example 1-1 as colorless liquid (32mmol, 7.1 g, 63% yield, trans:cis=2.1:1).

¹H NMR (400 MHz, CDCl₃, mixture of diasteromers) δ 3.85 - 3.38 (m, 1H), 2.63 - 2.46 (m, 1H), 2.44 - 2.14 (m, 4H), 2.08 - 1.65 (m, 6H), 1.45 - 1.04 (m, 3H), 0.99 - 0.95 (m, 4H), 0.93 - 0.74 (m, 3H) ppm. ¹³C NMR (101 MHz, CDCl₃, mixture of diasteromers) δ 198.0, 197.9, 173.0, 171.3, 116.2, 115.8, 82.0, 77.8, 37.4, 37.3, 32.1, 32.0, 31.7, 31.1, 29.2, 29.1, 28.9, 27.0, 26.1, 26.0, 21.1, 20.1, 18.1, 17.9, 17.8, 11.7, 10.4 ppm. GC/MS (EI): m/z (%): 222 (52) [M⁺], 207 (16), 193 (100), 179 (11), 153 (46), 137 (75), 125 (42), 91 (27), 79 (37), 67 (24), 55 (67).

### Odor description: woody, green, rooty, vetiver, slightly ambery powdery, orris isoraldéïne, peppery

The tans/cis isomers were isolated from 4-ethyl-2-isopropyl-2,3,4,6,7,8-hexahydro-5H-chromen-5-one via silica gel chromatography (Heptane: MTBE=20:1).

### Trans-4-ethyl-2-isopropyl-2,3,4,6,7,8-hexahydro-5H-chromen-5-one

¹H NMR (400 MHz, CDCl₃) δ 3.73 (dd, *J* = 11.9, 5.6 Hz, 1H), 2.61 - 2.49 (m, 1H), 2.43 - 2.27 (m, 4H), 2.03 - 1.64 (m, 5H), 1.43 - 1.35 (m, 1H), 1.18 - 1.04 (m, 1H), 1.03 - 0.95 (m, 6H), 0.91 (t, *J* = 7.4 Hz, 3H). ¹³C NMR (101 MHz, CDCl₃) δ 197.8, 171.2, 115.7, 77.7, 37.2, 32.1, 29.1, 28.8, 26.9, 25.9, 21.0, 18.1, 17.7, 11.6. GC/MS (EI): m/z (%): 222 (50) [M⁺], 207 (16), 193 (100), 151 (25), 137 (47), 125 (31), 55 (25).

### Cis-4-ethyl-2-isopropyl-2,3,4,6,7,8-hexahydro-5H-chromen-5-one

¹H NMR (400 MHz, CDCl₃) δ 3.50 (dd, *J* = 11.4, 5.6 Hz, 1H), 2.61 - 2.48 (m, 1H), 2.46 - 2.15 (m, 4H), 2.07 - 1.79 (m, 6H), 1.40 - 1.21 (m, 2H), 1.04 - 0.93 (m, 6H), 0.82 (t, *J* = 7.4 Hz, 3H) ppm. ¹³C NMR (101 MHz, CDCl₃) δ 197.9, 172.9, 116.1, 81.9, 37.4, 32.0, 31.6, 31.1, 29.0, 26.0, 20.0, 18.1, 17.8, 10.3 ppm. GC/MS (EI): m/z (%): 222 (53) [*M*⁺], 207 (6), 193 (75), 153 (100), 137 (52), 125 (36), 55 (28).

### Example 1-20: 4-ethyl-2-(prop-1-en-2-yl)-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol) and prop-1-en-2-ylmagnesium bromide (82 mL, 41 mmol, 0.5 M) according to the procedure of example 1-1 as colorless liquid (14 mmol, 3.0 g, 67% yield, trans:cis=3.3:1).

¹H NMR (400 MHz, CDCl₃, mixture of diasteromers) δ 5.19 - 4.87 (m, 2H), 4.48 - 4.06 (m, 1H), 2.65 - 2.51 (m, 1H), 2.48 - 2.19 (m, 4H), 2.14 - 1.69 (m, 7H), 1.65 - 1.05 (m, 2H), 1.00 - 0.76 (m, 3H) ppm. ¹³C NMR (101 MHz, CDCl₃, mixture of diasteromers) δ 197.8, 197.7, 172.3, 170.7, 143.6, 143.4, 116.1, 115.6, 112.7, 112.5, 80.1, 76.2, 37.3, 37.2, 33.3, 31.6, 29.3, 29.0, 28.8, 28.6, 26.9, 25.9, 21.0, 20.0, 18.3, 18.0, 11.7, 10.4 ppm. GC/MS (EI): m/z (%): 220 (86) [*M*⁺], 205 (4), 191 (100), 177 (23), 164 (29), 151 (45), 137 (50), 55 (31).

### Odor description: green, woody, peppery

### Example 1-21: 2-cyclopropyl-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 26mmol) and cyclopropylmagnesium bromide (102 mL, 51mmol, 0.5 M) according to the procedure of example 1-1 as colorless liquid (16mmol, 3.5 g, 62% yield, trans:cis=1:1).

¹H NMR (400 MHz, CDCl₃, mixture of diasteromers) δ 3.22 - 2.80 (m, 1H), 2.47 - 2.33 (m, 1H), 2.31 - 1.09 (m, 10H), 0.92 - 0.82 (m, 1H), 0.81 - 0.64 (m, 3H), 0.56 - 0.05 (m, 4H) ppm.¹³C NMR (101 MHz, CDCl₃, mixture of diasteromers) δ = 197.5, 197.4, 172.2, 170.7, 115.8, 115.4, 81.5, 77.7, 37.1, 37.0, 34.1, 31.3, 29.5, 29.1, 28.9, 28.7, 26.8, 25.9, 20.9, 19.9, 15.1, 14.9, 11.5, 10.2, 3.2, 1.7, 1.6 ppm. GC/MS (EI): m/z (%): 220 (13) [*M*⁺], 191 (52), 177 (11), 153 (41), 125 (57), 95 (21), 77 (22), 67 (100), 55 (53).

### Odor description: green, fatty, woody, peppery

### Example 1-22: 4-ethyl-2-(prop-1-en-1-yl)-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 26mmol) and prop-1-en-1-ylmagnesium bromide (51 mL, 51mmol, 1.0 M) according to the procedure of example 1 as colorless liquid (9.9 mmol, 2.2 g, 39% yield, trans:cis=1.3:1).

¹H NMR (400 MHz, CDCl₃, mixture of isomers) δ 5.85 - 5.56 (m, 1H), 5.55 - 5.30 (m, 1H), 4.83 - 4.07 (m, 1H), 2.66 - 2.40 (m, 1H), 2.40 - 2.10 (m, 4H), 2.05 - 1.56 (m, 7H), 1.54 - 1.34 (m, 1H), 1.33 - 0.95 (m, 1H), 0.94 - 0.65 (m, 3H) ppm. ¹³C NMR (101 MHz, CDCl₃, mixture of isomers) δ 197.8, 197.7, 172.3, 172.1, 170.8, 170.6, 130.0, 129.7, 129.6, 129.3, 129.2, 129.1, 128.4, 128.3, 116.1, 115.5, 115.4, 77.7, 74.0, 73.1, 69.2, 37.3, 37.2, 34.7, 34.5, 31.2, 31.1, 30.2, 30.0, 29.3, 29.2, 29.1, 28.9, 27.1, 27.0, 26.1, 26.0, 21.0, 20.1, 17.8, 13.4, 13.3, 11.8, 11.7, 10.4 ppm. GC/MS (EI): m/z (%): 220 (61) [*M*⁺], 205 (7), 191 (66), 177 (11), 165 (100), 153 (21), 137 (48), 68 (78).

### Odor description: woody, fruity, powdery, isoraldéïne orris, peppery

### Example 1-23: 2-allyl-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 26 mmol) and allylmagnesium bromide (51mLI, 51mmol, 1.0 M) according to the procedure of example 1-1 as colorless liquid (7.1 mmol, 1.6 g, 28% yield, trans:cis=1.3:1).

¹H NMR (400 MHz, CDCl₃, mixture of diasteromers) δ 5.93 - 5.49 (m, 1H), 5.26 - 4.88 (m, 2H), 4.18 - 3.36 (m, 1H), 2.50 - 2.08 (m, 7H), 2.03 - 1.55 (m, 4H), 1.40 - 0.94 (m, 2H), 0.88 - 0.66 (m, 3H) ppm. ¹³C NMR (101 MHz, CDCl₃, mixture of diasteromers) δ 197.8, 197.7, 172.4, 170.8, 133.3, 133.3, 117.9, 117.8, 116.1, 115.6, 72.6, 39.5, 39.4, 37.3, 37.2, 33.8, 31.2, 29.2, 29.0, 28.9, 28.7, 26.9, 25.9, 21.0, 20.0, 11.6, 10.3 ppm. GC/MS (EI): m/z (%): 220 (45) [M⁺], 205 (9), 191 (59), 177 (26), 153 (100), 137 (31), 125 (98), 55 (34).

### Odor description: woody, peppery, green

### Example 1-24: 4-ethyl-2-(2-methylprop-1-en-1-yl)-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol) and (2-methylprop-1-en-1-yl)magnesium bromide (82 mL, 41 mmol, 0.5 M) according to the procedure of example 1-1 as colorless liquid (13 mmol, 3.0 g, 63% yield, trans:cis=3.5:1).

¹H NMR (400 MHz, CDCl₃, mixture of diasteromers) δ 5.95 - 5.73 (m, 1H), 5.19 - 4.86 (m, 2H), 4.11 - 3.61 (m, 1H), 2.64 - 2.47 (m, 1H), 2.43 - 2.11 (m, 6H), 2.04 - 1.59 (m, 6H), 1.51 - 1.00 (m, 2H), 0.96 - 0.74 (m, 3H) ppm. ¹³C NMR (101 MHz, CDCl₃, mixture of diasteromers) δ 197.7, 172.4, 170.9, 137.7, 137.6, 123.8, 123.6, 116.0, 115.4, 74.4, 70.4, 37.3, 37.1, 34.9, 31.2, 30.2, 29.3, 29.1, 28.9, 27.0, 26.0, 25.7, 21.0, 20.0, 18.3, 18.2, 11.7, 10.3 ppm. GC/MS (EI): m/z (%): 234 (43) [*M*⁺], 205 (29), 191 (7), 177 (9), 165 (76), 82 (100), 67 (57).

### Odor description: green, grapefruit, bell pepper, woody.

### Example 1-25: 4-ethyl-2-isobutyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol) and isobutylmagnesium bromide (51 mL, 41mmol, 0.8 M) according to the procedure of example 1-1 as colorless liquid (16mmol, 3.8 g, 79% yield, trans:cis=2.5:1).

¹H NMR (400 MHz, CDCl₃, mixture of diasteromers) δ 4.17 - 3.68 (m, 1H), 2.63 - 2.45 (m, 1H), 2.42 - 2.17 (m, 4H), 2.07 - 1.57 (m, 6H), 1.46 - 1.02 (m, 3H), 0.99 - 0.75 (m, 9H) ppm. ¹³C NMR (101 MHz, CDCl₃, mixture of diasteromers)δ 197.9, 197.8, 172.7, 171.0, 116.1, 115.6, 75.6, 71.6, 44.4, 44.2, 37.4, 37.2, 35.0, 31.4, 30.1, 29.3, 29.1, 28.9, 27.1, 26.0, 24.4, 24.3, 23.1, 23.0, 22.4, 22.3, 21.0, 20.0, 11.8, 10.3 ppm. GC/MS (EI): m/z (%): 236 (70) [M⁺], 221 (10), 207 (100), 193 (71), 165 (20), 151 (96), 139 (35), 125 (53), 55 (23).

### Odor description: green, fruity, woody, cedarwood, peppery

### Example 1-26: 2-(sec-butyl)-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol) and sec-butylmagnesium bromide (41 ml, 41 mmol, 1.0 M) according to the procedure of example 1-1 as colorless liquid (15mmol, 3.6 g, 75% yield, trans:cis=2.8:1).

¹H NMR (400 MHz, CDCl₃, mixture of diasteromers) δ 3.92 - 3.54 (m, 1H), 2.61 - 2.46 (m, 1H), 2.44 - 2.17 (m, 4H), 2.06 - 1.88 (m, 2H), 1.87 - 1.65 (m, 2H), 1.64 - 1.17 (m, 4H), 1.16 - 1.02 (m, 1H), 1.00 - 0.77 (m, 9H) ppm. ¹³C NMR (101 MHz, CDCl₃, mixture of diasteromers) δ 197.8, 197.7, 173.1, 173.0, 171.3, 171.2, 116.1, 115.7, 115.6, 80.8, 80.6, 76.6, 76.4, 38.6, 38.6, 38.4, 38.3, 37.4, 37.2, 31.8, 31.6, 31.4, 30.5, 29.3, 29.1, 29.0, 28.8, 28.7, 26.9, 26.1, 26.0, 25.2, 25.1, 25.0, 24.9, 21.0, 20.0, 14.3, 14.2, 14.1, 14.0, 11.8, 11.7, 11.6, 11.5, 11.4, 11.3, 10.4, 10.3 ppm. GC/MS (EI): m/z (%): 236 (48) [*M*⁺], 221 (7), 207 (100), 189 (5), 179 (9), 165 (10), 153 (41), 137 (27), 125 (30), 109 (7), 95 (8), 79 (12), 67 (7), 55 (18).

### Odor description: woody, fruity powdery, isoraldéïne orris, peppery.

### Example 1-27: 2-(but-2-en-2-yl)-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 20mmol) and but-2-en-2-ylmagnesium bromide (82 mL, 41 mmol, 0.5 M) according to the procedure of example 1-1 as colorless liquid (14 mmol, 3.2 g, 67% yield, trans:cis=2.3:1).

¹H NMR (400 MHz, CDCl₃, mixture of diasteromers) δ 5.60 - 5.23 (m, 1H), 4.92 - 4.20 (m, 1H), 2.53 (m, 1H), 2.39 - 2.10 (m, 4H), 2.00 - 1.40 (m, 11H), 1.28 - 1.00 (m, 1H), 0.94 - 0.68 (m, 3H) ppm. ¹³C NMR (101 MHz, CDCl₃, mixture of diasteromers) δ 197.8, 197.7, 172.9, 171.1, 171.0, 134.2, 133.9, 123.4, 123.1, 122.9, 116.1, 115.5, 115.4, 78.5, 75.3, 71.1, 37.3, 37.2, 32.8, 31.6, 29.4, 29.1, 28.8, 28.3, 27.5, 27.0, 26.9, 26.1, 21.0, 20.0, 18.1, 17.9, 13.2, 12.9, 12.7, 11.8, 11.7, 11.5, 10.4 ppm. GC/MS (EI): m/z (%): 234 (59) [*M*⁺], 205 (46), 191 (29), 177 (23), 165 (95), 151 (26), 137 (43), 82 (90), 67 (100).

### Odor description: green,coniferous, terpenic, woody, tobacco, fiber, gurjum,peppery

### Example 1-28: 2-(but-3-en-2-yl)-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (3.0 g, 15mmol) and but-3-en-2-ylmagnesium bromide (34mL, 31 mmol, 0.9 M) according to the procedure of example 1-1 as colorless liquid (2.2 mmol, 0.5 g, 15% yield, trans:cis=1.3:1).

¹H NMR (400 MHz, CDCl₃, mixture of diasteromers) δ 5.85 - 5.52 (m, 1H), 5.13 - 4.85 (m, 2H), 3.90 - 3.42 (m, 1H), 2.53 - 2.08 (m, 6H), 2.03 1.55 (m, 4H), 1.43 - 1.11 (m, 2H), 1.10 - 0.94 (m, 3H), 0.88 - 0.68 (m, 3H) ppm. ¹³C NMR (101 MHz, CDCl₃, mixture of diasteromers)δ 197.9, 197.8, 172.8, 172.7, 171.1, 171.0, 139.5, 139.4, 139.1, 139.1, 116.2, 116.1, 115.8, 115.7, 115.6, 115.6, 80.4, 80.3, 76.3, 76.2, 42.1, 42.0, 41.7, 41.6, 37.4, 37.2, 31.7, 31.5, 31.4, 31.3, 29.1, 29.0, 28.8, 28.7, 26.9, 26.8, 26.6, 26.1, 26.0, 25.9, 21.0, 20.0, 15.5, 15.4, 15.3, 11.7, 11.6, 10.3, 10.2 ppm. GC/MS (EI): m/z (%): 234 (57) [M⁺], 219 (10), 205 (60), 177 (68), 163 (22), 153 (100), 137 (40), 125 (76), 81 (72), 55 (64).

### Odor description: woody, tobacco, cedarwood

### Example 1-29: 2-(tert-butyl)-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 25.5 mmol, 1.0 equiv) and tert-butylmagnesium chloride (51mL, 51mmol, 1.0 M) according to the procedure of example 1-1 as colorless liquid (16 mmol, 3.7 g, 61% yield).

¹H NMR (400 MHz, CDCl*3*, mixture of diasteromers) δ 3.66 - 3.32 (m, 1H), 2.64 - 2.48 (m, 1H), 2.44 - 2.15 (m, 4H), 2.10 - 1.64 (m, 4H), 1.43 - 1.04 (m, 2H), 0.97 (s, 9H), 0.93 - 0.77 (m, 3H) ppm. ¹³C NMR (101 MHz, CDCl₃, mixture of diasteromers) δ 197.6, 197.5, 173.0, 171.2, 115.9, 115.5, 84.6, 80.3, 37.2, 37.1, 33.5, 33.4, 31.9, 29.2, 29.0, 28.8, 28.6, 26.7, 25.9, 25.6, 25.5, 23.5, 20.9, 19.9, 11.5, 10.2 ppm. GC/MS (EI): m/z (%): 236 (21) [*M*⁺], 207 (43), 189 (15), 151 (43), 95 (21), 79 (31), 57 (100).

### Odor description: green, woody

### Example 1-30: 2-butyl-4-ethyl-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 25mmol) and butylmagnesium bromide (64 mL, 51mmol, 0.8 M,) according to the procedure of example 1-1 as colorless liquid (14mmol, 3.4 g, 57% yield, trans:cis=2.5:1).

¹H NMR (400 MHz, CDCl₃, mixture of diasteromers) δ 3.98 - 3.52 (m, 1H), 2.54 - 2.39 (m, 1H), 2.35 - 2.10 (m, 4H), 2.03 - 0.94 (m, 12H), 0.90 - 0.63 (m, 6H) ppm. ¹³C NMR (101 MHz, CDCl₃, mixture of diasteromers) δ 197.8, 197.7, 172.6, 171.0, 116.1, 115.6, 77.3, 73.3, 37.3, 37.2, 35.0, 34.8, 34.5, 31.4, 29.5, 29.3, 29.0, 28.9, 27.3, 27.2, 27.0, 26.0, 22.6, 22.5, 21.0, 20.0, 14.0, 13.9, 11.7, 10.3 ppm. GC/MS (EI): m/z (%): 236 (41) [*M*⁺], 221 (8), 207 (100), 193 (13), 165 (13), 153 (22), 125 (40), 55 (14).

### Odor description: green, cedarwood

### Example 1-31: 4-ethyl-2-(2-methylallyl)-2,3,4,6,7,8-hexahydro-5H-chromen-5-one

The compound was obtained from 4-ethyl-2-hydroxy-2,3,4,6,7,8-hexahydro-5H-chromen-5-one (5.0 g, 25mmol) and (2-methylallyl)magnesium bromide (64 mL, 51mmol, 0.8 M) according to the procedure of example 1-1 as colorless liquid (6.8 mmol, 1.6 g, 27% yield, trans:cis=1.1:1).

¹H NMR (400 MHz, CDCl₃, mixture of diasteromers) δ 5.08 - 4.66 (m, 2H), 4.25 - 3.70 (m, 1H), 2.70 - 2.14 (m, 7H), 2.13 - 1.64 (m, 7H), 1.47 - 1.02 (m, 2H), 0.98 - 0.70 (m, 3H). ¹³C NMR (101 MHz, CDCl₃, mixture of diasteromers) δ 197.8, 197.7, 172.5, 170.8, 141.3, 141.2, 116.2, 115.7, 113.2, 75.6, 71.8, 43.5, 43.4, 37.3, 37.2, 34.2, 31.3, 29.3, 29.2, 29.0, 28.8, 27.0, 26.0, 22.9, 22.8, 21.0, 20.0, 11.7, 10.3. GC/MS (EI): m/z (%): 234 (45) [*M*⁺], 219 (6), 205 (44), 191 (12), 177 (100), 153 (42), 81 (57), 67 (20), 55 (38).

### Odor description: green, woody

### Example 1-32: 2-ethyl-4-propyl-2,3,4,6,7,8-hexahydro-5H-chromen-5-one

The compound was obtained from 2-hydroxy-4-propyl-2,3,4,6,7,8-hexahydro-5H-chromen-5-one (10g, 48 mmol) and ethylmagnesium bromide (48 mL, 95mmol, 2.0 M) according to the procedure of example 1-1 as colorless liquid (40 mmol, 8.8 g, 83% yield).

¹H NMR (400 MHz, CDCl₃, mixture of diasteromers) δ 4.07 - 3.58 (m, 1H), 2.76 - 2.54 (m, 1H), 2.50 - 2.17 (m, 4H), 2.01 - 1.78 (m, 3H), 1.75 - 1.52 (m, 3H), 1.46 - 1.18 (m, 4H), 1.04 - 0.83 (m, 6H) ppm. ¹³C NMR (101 MHz, CDCl₃, mixture of diasteromers) δ = 197.9, 197.8, 172.4, 171.0, 116.5, 115.7, 98.7, 78.5, 74.4, 37.4, 37.2, 36.7, 36.0, 34.6, 30.2, 29.7, 29.0, 28.9, 28.2, 28.0, 27.4, 21.0, 20.3, 20.0, 19.5, 14.3, 14.1, 9.5, 9.4. GC/MS (EI): m/z (%): 222 (14) [*M*⁺], 193 (1), 179 (100), 151 (10), 137 (7), 125 (15).

### Odor description: green, woody, peppery, fruity

### Example 1-33: 4-isopropyl-2-methyl-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 2-hydroxy-4-isopropyl-2,3,4,6,7,8-hexahydro-5H-chromen-5-one was obtained from reaction of Cyclohexane-1,3-dione (25g, 223mmol) and 4-methylpent-2-enal (22 g, 223mmol) as white solid (166mmol, 35g, 75% yield). GC/MS (EI): m/z (%): 210 (11) [*M*⁺], 192 (4), 167 (100), 151 (35), 139 (75), 55 (25).
2. According to the procedure of example 1-1, 4-isopropyl-2-methyl-2,3,4,6,7,8-hexahydro-5H-chromen-5-one was obtained from 2-hydroxy-4-isopropyl-2,3,4,6,7,8-hexahydro-5H-chromen-5-one (5.0 g, 20mmol) and methylmagnesium bromide (14 mL, 40mmol, 3.0 M) colorless liquid (14mmol, 3.0 g, 71% yield, trans:cis=1:1).

¹H NMR (400 MHz, CDCl₃, mixture of diasteromers) δ 4.23 - 3.74 (m, 1H), 2.72 - 2.52 (m, 1H), 2.50 - 2.08 (m, 5H), 1.97 - 1.04 (m, 7H), 0.96 - 0.36 (m, 6H) ppm.¹³C NMR (101 MHz, CDCl₃, mixture of diasteromers) δ = 198.0, 197.9, 173.3, 171.1, 115.7, 113.9, 73.5, 70.6, 64.8, 37.4, 37.2, 35.5, 32.8, 32.2, 31.6, 31.0, 30.3, 29.9, 29.3, 29.2, 29.1, 26.4, 23.2, 21.4, 21.0, 20.9, 20.8, 20.7, 20.6, 20.5, 20.3, 20.0, 19.3, 15.0 ppm.

### Odor description: green, woody, ambery, peppery.

### Example 1-34: 2-ethyl-3,4-dimethyl-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 2-hydroxy-3,4-dimethyl-2,3,4,6,7,8-hexahydro-5H-chromen-5-one was obtained from reaction of cyclohexane-1,3-dione (20g, 178mmol) and 2-methylbut-2-enal (18g, 214mmol) as light yellow solid (23mmol, 4.5 g, 13% yield). GC/MS (EI): m/z (%): 196 (32) [*M*⁺], 177 (5), 167 (21), 153 (12), 139 (100), 55 (16).
2. According to the procedure of example 1-1, 2-ethyl-3,4-dimethyl-2,3,4,6,7,8-hexahydro-5H-chromen-5-one was obtained from 2-hydroxy-3,4-dimethyl-2,3,4,6,7,8-hexahydro-5H-chromen-5-one (5.0 g, 26 mmol) and ethylmagnesium bromide (51mL, 51mmol, 1.0 M) as colorless liquid (9.4 mmol, 2.0 g, 37% yield).

¹³C NMR (101 MHz, CDCl₃, mixture of isomers) 198.6, 198.3, 198.0, 197.7, 171.6, 171.2, 170.7, 170.6, 117.4, 117.2, 115.4, 114.7, 83.1, 82.5, 78.0, 77.0, 39.7, 37.6, 37.3, 37.0, 34.9, 34.9, 33.4, 32.5, 31.0, 30.5, 28.8, 28.7, 28.6, 28.6, 27.8, 25.1, 25.0, 24.9, 24.5, 21.5, 21.1, 21.1, 20.3, 20.1, 19.0, 15.8, 15.7, 14.7, 13.8, 12.4, 10.5, 10.1, 8.8, 8.6, 7.5 ppm. GC/MS (EI): m/z (%): 208 (85) [*M*⁺], 193 (91), 179 (65), 165 (94), 151 (45), 139 (73), 123 (20), 108 (24), 90 (39), 82 (67), 55 (100).

### Odor description: fruity peppery woody

### Example 1-35: 4-ethyl-2,3-dimethyl-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 4-ethyl-2-hydroxy-3-methyl-2,3,4,6,7,8-hexahydro-5H-chromen-5-one was obtained from reaction of cyclohexane-1,3-dione (60 g, 535 mmol and 2-methylpent-2-enal (63g, 642mmol) as white solid (285mmol, 60g, 53% yield).
   ¹³C NMR (101 MHz, CDCl₃, mixture of isomers) δ = 198.9, 198.8, 198.1, 169.7, 169.2, 168.3, 128.9, 115.8, 114.6, 113.9, 98.5, 97.9, 97.5, 94.6, 77.3, 49.4, 38.0, 37.2, 37.2, 37.0, 36.9, 36.0, 34.4, 33.3, 33.2, 32.4, 28.8, 28.5, 27.0, 26.9, 26.9, 23.7, 22.9, 21.0, 20.8, 20.6, 20.4, 17.0, 13.6, 12.7, 11.5, 11.3, 10.6, 8.4 ppm.
2. According to the procedure of example 1-1, 4-ethyl-2,3-dimethyl-2,3,4,6,7,8-hexahydro-5H-chromen-5-one was obtained from 4-ethyl-2-hydroxy-3-methyl-2,3,4,6,7,8-hexahydro-5H-chromen-5-one (5.0 g, 24 mmol) and methylmagnesium chloride (48 mL, 48 mmol, 1.0 M) as colorless liquid (14mmol, 2.9 g, 59% yield).

¹³C NMR (101 MHz, CDCl₃, mixture of isomers) δ 198.7, 198.5, 198.1, 197.7, 173.0, 171.8, 170.5, 170.4, 116.1, 115.2, 114.8, 113.9, 78.3, 77.5, 75.1, 71.5, 38.2, 37.9, 37.7, 37.4, 37.3, 37.2, 37.1, 37.0, 36.9, 33.2, 33.0, 31.7, 29.0, 28.9, 28.7, 27.6, 23.7, 21.6, 21.1, 20.9, 20.8, 20.2, 20.0, 19.6, 18.9, 18.0, 17.9, 16.3, 14.6, 13.1, 12.4, 11.8, 11.7, 8.5, 5.9 ppm. GC/MS (EI): *m*/*z* (%): 208 (26) [*M*⁺], 193 (14), 179 (100), 151 (30), 137 (69), 79 (40), 55 (61).

### Odor description: woody, tobacco, fiber, peppery, green, myrrh, mushroom

### Example 1-36: 2,4-diethyl-3-methyl-2,3,4,6,7,8-hexahydro-5H-chromen-5-one

The compound was obtained from 4-ethyl-2-hydroxy-3-methyl-2,3,4,6,7,8-hexahydro-5H-chromen-5-one (5.0 g, 24 mmol) and ethylmagnesium chloride (24 mL, 48 mmol, 2.0 M) according to the procedure of example 1-1 as colorless liquid (19 mmol, 4.2 g, 79% yield).

¹H NMR (400 MHz, CDCl₃, mixture of isomers) δ 3.92 - 3.33 (m, 1H), 2.66 - 1.04 (m, 12H), 1.03 - 0.59 (m, 9H) ppm.¹³C NMR (101 MHz, CDCl₃, mixture of isomers) δ = 198.6, 198.4, 198.0, 197.6, 172.9, 171.9, 170.6, 170.5, 116.0, 114.8, 114.1, 83.2, 82.8, 79.3, 38.0, 37.9, 37.7, 37.4, 37.3, 37.2, 36.9, 34.1, 33.8, 33.2, 31.1, 29.8, 28.9, 28.8, 28.6, 27.6, 25.5, 25.2, 25.1, 24.9, 23.7, 21.9, 21.1, 20.9, 20.8, 20.2, 19.9, 16.4, 14.4, 13.0, 12.5, 11.7, 11.6, 10.2, 10.1, 9.1, 8.7, 8.5, 5.9 ppm. GC/MS (EI): m/z (%): 222 (56) [*M*⁺], 207 (11), 193 (100), 153 (76), 137 (57), 55 (28).

### Odor description: sweet, caramel, woody, guaïac, tobacco, cedarwood, peppery, green

### Example 1-37: 2-ethyl-4,7-dimethyl-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 2-hydroxy-4,7-dimethyl-2,3,4,6,7,8-hexahydro-5H-chromen-5-one was obtained from reaction of 5-methylcyclohexane-1,3-dione (50g, 396mmol) and but-2-enal (31 g, 436mmol) as white solid (285mmol, 60. g, 53% yield).
   ¹H NMR (400 MHz, CDCl₃, mixture of isomers) δ 5.76 - 5.15 (m, 2H), 3.02 - 2.69 (m, 1H), 2.63 - 1.63 (m, 7H), 1.47 - 0.92 (m, 6H) ppm. ¹³C NMR (101 MHz, CDCl₃, mixture of isomers) δ 198.8, 198.5, 169.7, 169.6, 116.2, 115.5, 115.3, 94.9, 93.9, 93.3, 93.2, 45.5, 45.4, 45.3, 45.0, 37.0, 36.8, 36.8, 36.4, 35.6, 34.2, 28.8, 28.6, 28.1, 27.6, 23.5, 22.8, 21.8, 21.1, 21.1, 21.0, 20.9, 20.8, 20.4, 20.0 ppm. GC/MS (EI): m/z (%): 196 (65) [*M*⁺], 181 (6), 167 (100), 153 (88), 69 (30).
2. 2-ethyl-4,7-dimethyl-2,3,4,6,7,8-hexahydro-5H-chromen-5-one was obtained from 2-hydroxy-4,7-dimethyl-2,3,4,6,7,8-hexahydro-5H-chromen-5-one (5.0 g, 26 mmol) and ethylmagnesium chloride (51mL, 51mmol, 1.0 M,) according to the procedure of example 1-1 as colorless liquid (16mmol, 3.4 g, 64% yield).
   ¹H NMR (400 MHz, CDCl₃, mixture of isomers) δ 4.16 - 3.57 (m, 1H), 2.87 - 2.55 (m, 1H), 2.51 - 1.24 (m,9), 1.21 - 0.84 (m, 9H) ppm.¹³C NMR (101 MHz, CDCl₃, mixture of isomers, main peaks) δ = 198.1, 197.7, 170.7, 170.6, 170.1, 116.0, 115.7, 115.6, 78.3, 74.4, 74.3, 45.7, 45.2, 37.7, 37.3, 37.2, 37.0, 36.9, 36.7, 33.6, 33.5, 28.8, 28.2, 28.1, 27.9, 27.8, 27.4, 25.8, 25.4, 22.7, 22.3, 21.3, 21.2, 20.9, 20.8, 20.5, 20.3, 20.1, 9.6, 9.5, 9.4 ppm. GC/MS (EI): m/z (%): 208 (95) [M⁺], 193 (99), 179 (24), 165 (100), 153 (83), 138 (28), 55 (25).

### Odor description: woody, fruity, allyl ionone, green

### Example 1-38: 4-methyl-2-propyl-2,3,4,6,7,8-hexahydro-5H-chromen-5-one and 2-ethyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (145 g, 796 mmol, 1.0 equiv) and ethylmagnesium chloride (80 mL, 159 mmol, 2.0 M, 0.2 equiv) and propylmagnesium chloride (716 mL, 1432 mmol, 2.0 M, 1.8 equiv) according to the procedure of example 1-1 as colorless liquid (692 mmol, 144g, 87% yield, contain 90% of 4-methyl-2-propyl-2,3,4,6,7,8-hexahydro-5H-chromen-5-one with trans/cis=2.3:1) ¹³C NMR (101 MHz, CDCl₃, mixture of isomers, main peaks) δ = 198.1, 172.0, 171.3, 117.3, 116.1, 74.3, 72.9, 63.4, 38.2, 37.4, 37.2, 37.1, 37.1, 34.1, 33.6, 29.0, 28.8, 28.7, 28.6, 28.2, 25.8, 22.6, 21.7, 21.1, 21.0, 20.9, 20.4, 20.4, 20.1, 18.4, 18.3, 14.0, 9.4 ppm.

### Odor description: spicy, peppery, woody, acet guayil, fruity, berry

### Example 1-39: 2-isopropyl-4-methyl-2,3,4,6,7,8-hexahydro-5H-chromen-5-one and 2-ethyl-4-methyl-2,3,4,6,7,8-hexahydro-5H-chromen-5-one

The compound was obtained from 2-hydroxy-4-methyl-2,3,4,6,7,8-hexahydro-5H-chromen-5-one (7.0 g, 38mmol, 1.0 equiv) and ethylmagnesium chloride (3.9 mL, 7.7 mmol, 2.0 M, 0.2 equiv) and isopropylmagnesium chloride (35mL, 69mmol, 2.0 M, 1.8 equiv) according to the process of example 1-1 as colorless liquid (31mmol, 6.5 g, 81% yield, contains 90% of 2-isopropyl-4-methyl-2,3,4,6,7,8-hexahydro-5H-chromen-5-one with trans/cis=2.2:1).

¹³C NMR (101 MHz, CDCl₃, mixture of isomers, main peaks) δ = 197.7, 172.0, 171.2, 117.2, 116.0, 81.8, 78.3, 77.5, 74.2, 37.6, 37.2, 37.1, 34.8, 33.5, 31.9, 31.8, 30.5, 28.9, 28.8, 28.7, 28.6, 28.1, 27.8, 25.9, 25.7, 22.5, 22.4, 21.0, 20.9, 20.3, 20.0, 18.0, 17.9, 17.7, 17.7, 9.3, 9.3 ppm.

### Odor description: violet, fruity, woody, peppery

### Example 1-40: 2-ethyl-4-(prop-1-en-1-yl)-2,3,4,6,7,8-hexahydro-5H-chromen-5-one

1. According to the synthetic procedure of 2-hydroxy-4-methyl-2,3,4,6,7,8-hexahydro-5H-chromen-5-one in example 1-1, 2-hydroxy-4-(prop-1-en-1-yl)-2,3,4,6,7,8-hexahydro-5H-chromen-5-one was obtained from reaction of cyclohexane-1,3-dione (5.0 g, 45 mmol, 1.0 equiv) and E,E-2,4-hexadienal (4.7 g, 49 mol, 1.1 equiv) as thick oil (45 mmol, 5.1 g, 55 % yield).
   2-hydroxy-4-(prop-1-en-1-yl)-2,3,4,6,7,8-hexahydro-5H-chromen-5-one- GC/MS (EI): m/z (%): 208 (23) [*M*⁺], 190 (5), 180 (31), 163 (61), 149 (100), 139 (83), 111 (55), 55 (60).
2. The compound was obtained from 2-hydroxy-4-(prop-1-en-1-yl)-2,3,4,6,7,8-hexahydro-5H-chromen-5-one (3.5 g, 17 mmol, 1.0 equiv) and ethylmagnesium bromide (34 mL, 34 mmol, 1.0 M, 2.0 equiv) according to the procedure of example 1-1 as colorless liquid (98.0 mmol, 0.41 g, 11% yield, trans:cis=1:1).

¹H NMR (400 MHz, CDCl₃) trans isomer: δ 5.53 - 5.38 (m, 1H), 5.34 - 5.19 (m, 1H), 3.96 - 3.84 (m, 1H), 3.36 - 3.33 (m, 1H), 2.51 - 2.26 (m, 4H), 2.04 - 1.91 (m, 2H), 1.80 - 1.46 (m, 7H), 0.99 (t, *J* = 7.4 Hz, 3H), cis isomer δ 5.53 - 5.36 (m, 2H), 3.80 - 3.73 (m, 1H), 3.24 - 3.14 (m, 1H), 2.44 - 2.20 (m, 4H), 2.03 - 1.46 (m, 9H), 0.98 (t, *J* = 7.5 Hz, 3H) ppm. ¹³C

NMR (101 MHz, CDCl₃) trans isomer δ = 197.4, 171.9, 133.5, 125.8, 112.9, 74.7, 37.2, 31.9, 30.1, 29.0, 28.0, 21.1, 17.9, 9.4, cis isomer δ = 197.4, 172.1, 133.9, 124.1, 115.0, 78.3, 37.3, 35.3, 33.1, 29.1, 27.9, 20.1, 18.0, 9.5 ppm. GC/MS (EI): m/z (%): trans isomer: 220 (42) [*M*⁺], 205 (15), 191 (100), 179 (41), 163 (35), 149 (54), 91(30), 55 (35); cis isomer: 220 (50) [*M*⁺], 205 (14), 191 (100), 179 (25), 163 (36), 149 (61), 91(28), 55 (32).

### Odor description: woody, peppery, green, fruity.

### Example 2-1: 2,4-diethyl-2,3,4,6,7,8-hexahydro-5H-chromen-5-one and 2-ethyl-4-methyl-2,3,4,6,7,8-hexahydro-5H-chromen-5-one

1. A mixture of cyclohexane-1,3-dione (240 g, 2.1 mol, 1.0 equiv), propionaldehyde (175g, 3.0 mol, 1.41 equiv), ethoxyethene (222g, 3.1 mol, 1.44 equiv) and L-proline (2.5 g, 21mmol, 0.01 equiv) in auto clave bottle was stirred at 135°C (oil temperature) for 16 hours under argon. The solvent was removed to get 503g as yellow liquid. The residue was distilled via kugelrohr distillation to give mixture of 2-ethoxy-4-ethyl-2,3,4,6,7,8-hexahydro-5H-chromen-5-one and 2-ethoxy-4-methyl-2,3,4,6,7,8-hexahydro-5H-chromen-5-one (459 g, contains 95% of 2-ethoxy-4-ethyl-2,3,4,6,7,8-hexahydro-5H-chromen-5-one, yield: 95%) as a light yellow oil.
   2-ethoxy-4-ethyl-2,3,4,6,7,8-hexahydro-5H-chromen-5-one (contain 5% of 2-ethoxy-4-methyl-2,3,4,6,7,8-hexahydro-5H-chromen-5-one): ¹³C NMR (101 MHz, CDCl₃, mixture, main peaks) 197.9, 197.7, 169.0, 168.4, 116.1, 115.5, 99.8, 99.3, 98.7, 65.1, 64.8, 64.7, 37.3, 37.2, 34.1, 30.1, 30.0, 29.4, 28.9, 28.7, 28.7, 26.9, 25.6, 20.9, 20.6, 20.5, 20.3, 15.2, 15.2, 11.6, 11.2 ppm.
2. A solution of 2-ethoxy-4-ethyl-2,3,4,6,7,8-hexahydro-5H-chromen-5-one and 2-ethoxy-4-methyl-2,3,4,6,7,8-hexahydro-5H-chromen-5-one (400g, 1.8 mol, 1.0 equiv) and acetic acid (268 g, 4.5 mol, 2.5 equiv) in THF (140 mL) and Water (420 mL) was stirred for 48 h under argon at 120°C and then cooled down and poured to the solution of NaHCO₃ (438 g, 5.2 mol, 2.9 equiv) in 2L water and then extracted by EA (500 mL*4), the organic layer was dried over MgSO₄ and the solvent was removed by rotary evaporation to get a solid, the solid was recrystallized to give 4-ethyl-2-hydroxy-2,3,4,6,7,8-hexahydro-5H-chromen-5-one one and 2-hydroxy-4-methyl-2,3,4,6,7,8-hexahydro-5H-chromen-5-one (317 g, contains 95% of 4-ethyl-2-hydroxy-2,3,4,6,7,8-hexahydro-5H-chromen-5-one, yield: 90%,) as white solid.
   4-ethyl-2-hydroxy-2,3,4,6,7,8-hexahydro-5H-chromen-5-one and 2-hydroxy-4-methyl-2,3,4,6,7,8-hexahydro-5H-chromen-5-one:¹³C NMR (101 MHz, CDCl₃, mixture of isomers, main peaks) δ 198.7, 198.5, 170.1, 169.7, 115.9, 115.3, 94.8, 93.3, 37.1, 37.0, 31.6, 31.3, 29.7, 29.3, 28.9, 28.8, 27.0, 25.7, 20.9, 20.3, 11.6, 11.1 ppm.
3. To a solution of 4-ethyl-2-hydroxy-2,3,4,6,7,8-hexahydro-5H-chromen-5-one and 2-hydroxy-4-methyl-2,3,4,6,7,8-hexahydro-5H-chromen-5-one (310g, 1.6 mol, 1.0 equiv) in THF (1.5 L) was added ethylmagnesium bromide (1.58 L, 2.0 M, 3.2 mol, 2.0 equiv) at 10~15°C, and the mixture was stirred for 16 hours under argon atmosphere at rt. The mixture was cooled by ice bath, and hydrogen chloride (1.16 L, 3.0 M, 3.5 mol, 2.2 equiv) was added slowly, the temperature was controlled below 20°C, then the mixture was stirred for 16h at rt. The organic layer was collected, and the water phase was extracted with EA (500mL*4), then the organic layers were combined and washed by NaHCO₃ solution and NaCl solution. The organic phase was dried with MgSO₄, and filtered, the solvent was removed by rotary evaporation to give the crude oil. The oil was purified by distilled via kugelrohr distillation to give product 2,4-diethyl-2,3,4,6,7,8-hexahydro-5H-chromen-5-one and 2-ethyl-4-methyl-2,3,4,6,7,8-hexahydro-5H-chromen-5-one (301g, trans/cis=1.75:1, contain 95% of 2,4-diethyl-2,3,4,6,7,8-hexahydro-5H-chromen-5-one yield: 92%).

¹H NMR (400 MHz, CDCl₃, mixture of isomers) δ 3.98 - 3.58 (m, 1H), 2.61 - 2.48 (m, 1H), 2.44 - 2.16 (m, 4H), 2.11 - 1.78 (m, 4H), 1.75 - 1.23 (m, 4H), 1.21 - 1.06 (m, 1H), 1.04 - 0.96 (m, 3H), 0.95 - 0.79 (m, 3H) ppm. ¹³C NMR (101 MHz, CDCl₃, mixture of isomers, main peaks) δ 198.0, 197.9, 172.7, 171.1, 116.1, 115.7, 78.5, 74.5, 37.4, 37.3, 34.0, 31.4, 29.3, 29.1, 29.0, 28.9, 28.8, 28.2, 28.0, 27.0, 26.1, 21.1, 20.0, 11.7, 10.4, 9.5, 9.5 ppm.

### Odor description: fruity woody, violet, cedarwood

### Example 2-2: 2,4-diethyl-3,4,6,7-tetrahydrocyclopenta[b]pyran-5(2H)-one

1. According to the procedure of example 2-1, 2-ethoxy-4-ethyl-3,4,6,7-tetrahydrocyclopenta[b]pyran-5(2H)-one was obtained from cyclopentane-1,3-dione (20 g, 204mmol, 1.0 equiv) and propionaldehyde (36g, 612 mmol, 3.0 equiv) and ethoxyethene (16g, 224mmol, 1.2 equiv) as colorless liquid, further puritication via silica gel chromatography to give the pure product (88mmol, 19 g, 43% yield, trans:cis=1:1).
   2-ethoxy-4-ethyl-3,4,6,7-tetrahydrocyclopentalblpyran-5(2H)-one: ¹H NMR (400 MHz, CDCl₃, mixture of diasteromers) δ 5.41 - 5.05 (m, 1H), 4.10 - 3.84 (m, 1H), 3.77 - 3.48 (m, 1H), 2.70 - 2.30 (m, 5H), 2.19 - 1.85 (m, 2H), 1.83 - 1.62 (m, 1H), 1.57 - 0.77 (m, 7H) ppm. ¹³C NMR (101 MHz, CDCl₃, mixture of diasteromers) δ = 203.8, 203.7, 181.8, 181.7, 118.2, 118.1, 102.6, 100.7, 65.2, 65.1, 33.5, 33.4, 31.1, 30.8, 29.4, 27.7, 26.0, 25.9, 25.0, 24.5, 15.1, 15.0, 11.1, 11.0 ppm.GC/MS (EI): m/z (%): 210 (27) [*M*⁺], 195 (1), 181 (100), 153 (19), 138 (30), 125 (48), 55 (14).
2. According to the procedure of example 2-1, 4-ethyl-2-hydroxy-3,4,6,7-tetrahydrocyclopenta[b]pyran-5(2H)-one was obtained from hydrolysis of 2-ethoxy-4-ethyl-3,4,6,7-tetrahydrocyclopenta[b]pyran-5(2H)-one (18 g, 86mmol) as white solid (13 g, 69mmol, yield:81%). ¹H NMR (400 MHz, CDCl₃) δ 5.71 - 5.49 (m, 2H), 2.83 - 1.17 (m, 12H), 0.97 - 0.79 (m, 3H) ppm. ¹³C NMR (101 MHz, CDCl₃, main peak) δ 204.8, 183.2, 118.1, 95.7, 33.5, 31.8, 27.9, 26.2, 25.6, 11.2 ppm.
3. According to the process of example 2-1, the title compound was obtained from 4-ethyl-2-hydroxy-3,4,6,7-tetrahydrocyclopenta[b]pyran-5(2H)-one (4.0 g, 22mmol) and ethylmagnesium bromide (22 mL, 44 mmol, 2.0M) according to the process of example 2-1 as colorless liquid (13 mmol, 2.5 g, 59% yield, trans:cis=1:1).

¹³C NMR (101 MHz, CDCl₃, mixture of diasteromers) δ 203.9, 203.8, 184.8, 184.1, 118.7, 117.6, 81.4, 77.6, 32.8, 31.6, 29.7, 29.0, 28.1, 27.8, 26.3, 23.8, 11.6, 10.5, 9.6, 9.5 ppm.

### Odor description: woody, peppery.

### Example 2-3: 4-ethyl-2-propyl-3,4,6,7-tetrahydrocyclopenta[b]pyran-5(2H)-one

The title compound was obtained from 4-ethyl-2-hydroxy-3,4,6,7-tetrahydrocyclopenta[b]pyran-5(2H)-one (4.0 g, 22mmol) and propylmagnesium bromide (22 mL, 44mmol, 2.0 M) according to the procedure of example 2-1 as colorless liquid (7.7 mmol, 1.6 g, 35% yield, trans:cis=1:1).

¹H NMR (400 MHz, CDCl₃, mixture of diasteromers) δ 4.30 - 3.92 (m, 1H), 2.62 - 1.92 (m, 6H), 1.87 - 1.69 (m, 2H), 1.67 - 1.13 (m, 5H), 1.08 - 0.73 (m, 6H) ppm. ¹³C NMR (101 MHz, CDCl₃, mixture of diasteromers) δ = 203.8, 203.8, 184.8, 184.0, 118.6, 117.5, 79.9, 76.2, 37.2, 36.9, 33.5, 33.4, 33.3, 31.6, 30.2, 29.0, 26.9, 26.2, 25.9, 23.8, 18.4, 18.3, 13.9, 11.6, 10.4 ppm. GC/MS (EI): m/z (%): 208 (64) [*M*⁺], 193 (2), 179 (45), 139 (100), 125 (28), 111 (46), 55 (26).

### Odor description: fruity, woody

### Example 2-4: 2,2,4-trimethyl-2,3,4,6,7,8-hexahydro-5H-chromen-5-one

1. A mixture of cyclohexane-1,3-dione (10g, 89mmol, 1.0 equiv), acetaldehyde (5.9 g, 13mmol, 1.5 equiv) and 2-methoxyprop-1-ene (9.7 g, 13mmol, 1.5 equiv) in auto clave bottle was stirred at 135°C (oil temperature) for 16 h. The solvent was removed to get 19.2 g as yellow liquid. The residue was purified by column chromatography on silica gel (hexane:MTBE=3:1) to get 2-methoxy-2,4-dimethyl-2,3,4,6,7,8-hexahydro-5H-chromen-5-one (14.5 g, 69mmol, yield: 77%, trans:cis=1.2:1) as a light yellow oil.
   ¹H NMR (400 MHz, CDCl₃, mixture of diasteromers) δ 3.37 - 3.19 (m, 3H), 2.75 - 1.39 (m, 12H), 1.23 - 1.14 (m, 3H) ppm. ¹³C NMR (101 MHz, CDCl₃, mixture of diasteromers) δ 198.0, 197.9, 167.9, 167.8, 117.8, 116.2, 101.2, 99.9, 49.3, 49.2, 42.1, 38.5, 37.4, 37.3, 28.6, 22.9, 22.7, 22.5, 22.2, 20.7, 20.1, 19.8, 19.4 ppm.
2. To a solution of 2-mthoxy-2,4-dimethyl-2,3,4,6,7,8-hexahydro-5H-chromen-5-one (11g, 49mmol, 1.0 equiv) and acetic acid (30 g, 490mmol, 10 equiv) in THF (20 mL) and Water (60 mL) was stirred for 24 h under argon atmosphere at 120°C. The mixture was cooled down and added to the solution of NaHCO₃ and then extracted by EA (150 mL*4), the organic layer was dried with MgSO4 and filtered and solvent was removed by rotary evaporation to get solid, the solid was washed with solvent (PE:MTBE=20:1) 50mL*3 to give product 2-hydroxy-2,4-dimethyl-2,3,4,6,7,8-hexahydro-5H-chromen-5-one (9.2 g, 47 mmol, yield: 96%) as white solid.
   ¹H NMR (400 MHz, CDCl₃, mixture of diasteromers) δ 3.84 (d, *J* = 3.2 Hz, 1H), 3.31 - 2.64 (m, 1H), 2.51 - 2.02 (m, 5H), 1.98 - 1.74 (m, 3H), 1.64 - 1.44 (m, 3H), 1.23 (m, 3H)ppm. ¹³C NMR (101 MHz, CDCl₃, mixture of diasteromers) δ 198.4, 198.3, 169.0, 168.8, 116.5, 115.6, 99.3, 97.9, 49.2, 41.6, 39.5, 37.3, 37.2, 29.8, 29.2, 29.1, 28.1, 27.7, 24.5, 23.3, 22.2, 20.6, 20.2, 19.8, 19.6, 18.8 ppm. GC/MS (EI): m/z (%): 196 (18) [M⁺], 181 (1), 153 (100), 139 (56), 55 (21).
3. The title compound was obtained from 2-hydroxy-2,4-dimethyl-2,3,4,6,7,8-hexahydro-5H-chromen-5-one (2.5 g, 13 mmol) and methylmagnesium bromide (26 ml, 26 mmol, 1.0 M) according to the procedure of example 2-1 as colorless liquid (10mmol, 2.0 g, 81% yield).

¹H NMR (400 MHz, CDCl₃) δ 2.65 - 2.52 (m, 1H), 2.46 - 2.16 (m, 4H), 1.97 - 1.76 (m, 3H), 1.51 -1.39 (m, 1H), 1.36 (s, 3H), 1.19 (s, 6H) ppm. ¹³C NMR (101 MHz, CDCl₃) δ 197.9, 170.2, 115.6, 77.0, 43.0, 37.4, 29.5, 29.0, 24.2, 23.0, 20.2, 19.9 ppm. GC/MS (EI): m/z (%): 194 (42) [M⁺], 179 (14), 161 (8), 151 (100), 139 (54), 55 (13).

### Odor description: weak, woody, fruity, allyl ionone, green, cork.

### Example 3-1: 2,4-dimethyl-4,6,7,8-tetrahydro-5H-chromen-5-one

To the suspension of the solution of cyclohexane-1,3-dione (10.0 g, 89.0 mmol, 1.0 equiv) and pent-3-en-2-one (7.5 g, 89mmol, 1.0 equiv) in toluene (250 mL), zinc(II) chloride (9.7 g, 71mmol, 0.8 equiv) was added dropwise at rt. and stirred at 120°C for 36 h. The solvent was removed the residue was purified by column chromatography on silica gel (PE:MTBE=2:1) to give 2,4-dimethyl-4,6,7,8-tetrahydro-5H-chromen-5-one as light yellow liquid (20mmol, 3.6 g, 22% yield).

¹H NMR (400 MHz, CDCl₃) δ 4.78 (d, *J* = 4.1 Hz, 1H), 3.26 - 3.02 (m, 1H), 2.50 - 2.25 (m, 4H), 2.05 - 1.92 (m, 2H), 1.81 (s, 3H), 1.08 (d, *J* = 6.6 Hz, 3H) ppm. ¹³C NMR (101 MHz, CDCl₃) δ 198.3, 166.8, 145.5, 115.0, 105.9, 37.2, 27.7, 23.8, 23.6, 20.6, 18.5 ppm. GC/MS (EI): m/z (%): 178 (54) [*M*⁺], 163 (90), 135 (30), 121 (100), 107 (34), 91 (43), 77 (67), 65 (23), 55 (53).

### Odor description: woody, tobacco, cedarwood, peppery, green, honey, aromatic

### Example 3-2: 2,4-dimethyl-2,3,4,6,7,8-hexahydro-5H-chromen-5-one

To a solution of 2,4-dimethyl-4,6,7,8-tetrahydro-5H-chromen-5-one (1 g, 5.6 mmol) in Methanol (50 mL) was added 10 % Pd/C (0.1 g) and stirred under hydrogen atmosphere for 1h, the Pd/C is fillter off and the product is purified by chromatography to give 2,4-dimethyl-2,3,4,6,7,8-hexahydro-5H-chromen-5-one as colorless liquid (3.5 mmol, 0.63 g, 62 % yield).

¹H NMR (400 MHz, CDCl₃) δ 3.99 - 3.85 (m, 1H), 2.71 - 2.61 (m, 1H), 2.45 - 1.77 (m, 8H), 1.40 - 1.24 (m, 3H), 1.17 (d, *J =* 6.5 Hz, 3H) ppm. ¹³C NMR (101 MHz, CDCl₃) δ 198.0, 171.8, 117.2, 73.5, 40.1, 37.4, 29.0, 25.8, 20.9, 20.3, 20.1 ppm.

### Odor description: woody, peppery, green, fruity.

### Example 3-3: cis-2,4-diethyl-4,6,7,8-tetrahydro-5H-chromen-5-one

The compound was obtained from cyclohexane-1,3-dione (10g, 89mmol) and (E)-hept-4-en-3-one (10g, 89mmol) according to the procedure of example 3-1 as colorless liquid (9.4 mmol, 1.9 g, 10% yield).

¹H NMR (400 MHz, CDCl₃) δ 4.76 (d, *J* = 4.8 Hz, 1H), 3.18 (dd, *J* = 9.9, 4.4 Hz, 1H), 2.56 - 2.25 (m, 4H), 2.21 - 2.09 (m, 2H), 2.06 - 1.88 (m, 2H), 1.55 - 1.37 (m, 2H), 1.08 (t, *J* = 7.5 Hz, 3H), 0.78 (t, *J* = 7.5 Hz, 3H)ppm. ¹³C NMR (101 MHz, CDCl₃) δ 198.3, 167.9, 151.9, 113.1, 101.8, 37.3, 29.3, 28.3, 27.7, 25.7, 20.6, 11.2, 9.2 ppm. GC/MS (EI): m/z (%): 206 (1) [*M*⁺], 177 (100), 149 (6), 135 (19), (16).

### Odor description: woody, peppery, warm, animalic, honey

### Example 3-4: 2,3,4-trimethyl-4,6,7,8-tetrahydro-5H-chromen-5-one

The title compound was obtained from cyclohexane-1,3-dione (10.0 g, 89mmol) and 3-methylpent-3-en-2-one (9.6 g, 98mmol) according to the procedure of example 3-1 as colorless liquid (9.9 mmol, 1.9 g, 11% yield).

¹H NMR (400 MHz, CDCl₃) δ 3.13 - 2.86 (m, 1H), 2.58 - 2.22 (m, 4H), 2.06 - 1.91 (m, 2H), 1.83 (s, 3H), 1.66 (s, 3H), 1.06 (d, *J* = 5.6 Hz, 3H) ppm. ¹³C NMR (101 MHz, CDCl₃) δ = 198.2, 166.7, 140.3, 114.9, 111.3, 37.1, 28.5, 27.5, 20.7, 20.6, 15.5, 15.3 ppm. GC/MS (EI): m/z (%): 192 (4) [M⁺], 177 (100), 163 (1), 149 (3), 135 (4), 121 (5), 105 (2), 91 (9), 77 (9), 65 (3), 55 (6).

### Odor description: fruity, berry, sweet, maltol, woody.

### Example 4-1: (4R)-2-ethyl-4-methyl-2,3,4,6,7,8-hexahydro-5H-chromen-5-one

1. To a solution of cyclohexane-1,3-dione (50.0 g, 446 mmol, 1.0 equiv) and but-2-enal (34 g, 491 mmol, 1.1 equiv) in Dichloromethane (250 mL) was added (S)-2-(diphenyl((trimethylsilyl)oxy)methyl)pyrrolidine (15 g, 45 mmol, 0.1 equiv) in Dichloromethane (100 mL) at 0°C and stirred for 6 h under argon. The solvent was removed and gave crude oil. The residue was purified by column chromatography on silica gel (hexane:MTBE=5:1 to 1:1) to get *(4R)*-2-hydroxy-4-methyl-2,3,4,6,7,8-hexahydro-5H-chromen-5-one (66 g, 362 mmol, 81% yield) as a white solid.
   ¹H NMR (400 MHz, CDCl₃, mixture of diasteromers) δ 5.80 - 5.03 (m, 2H), 3.07 - 2.67 (m, 1H), 2.51 - 2.25 (m, 4H), 2.13 - 1.67 (m, 4H), 1.32 - 1.06 (m, 3H) ppm. ¹³C NMR (101 MHz, CDCl₃, mixture of diasteromers) δ = 198.8, 198.6, 170.0, 169.2, 116.5, 115.9, 94.4, 93.1, 37.1, 36.9, 35.7, 35.6, 28.8, 28.7, 23.1, 22.9, 21.0, 20.9, 20.4, 20.3 ppm. GC/MS (EI): m/z (%): 182 (72) [M⁺], 167 (6), 153 (99), 139 (100), 126 (24), 111 (25), 97 (16), 83 (29), 69 (25), 55 (40).
2. To a solution of (4R)-2-hydroxy-4-methyl-2,3,4,6,7,8-hexahydro-5H-chromen-5-one (5.0 g, 27mmol) in Tetrahydrofuran (100 mL) was added ethylmagnesium bromide (55ml, 55 mmol, 1.0 M) in Tetrahydrofuran (50 mL) at -10°C and stirred for 6 h under argon at rt. NH₄Cl aqueous solution was added, and the organic layer was concentrated and the residue was dissolved in toluene (100 mL) and 4-methylbenzenesulfonic acid (0.5 g, 2.7 mmol, 0.1 equiv) was added, the mixture was stirred for 16h at 100°C, The solvent was removed get 4.2 g as yellow liquid. The residue was purified by column chromatography on silica gel (hexane:MTBE=9:1) to get (4R)-2-ethyl-4-methyl-2,3,4,6,7,8-hexahydro-5H-chromen-5-one (3.3 g, 17mmol, yield: 62%) as a colorless liquid.

¹H NMR (400 MHz, CDCl₃, mixture of diasteromers) δ 4.11 - 3.57 (m, 1H), 2.84 - 2.55 (m, 1H), 2.47 - 2.16 (m, 4H), 2.11 - 1.24 (m, 6H), 1.20 - 1.05 (m, 3H), 1.04 - 0.94 (m, 3H) ppm. ¹³C NMR (101 MHz, CDCl₃, mixture of diasteromers) δ 197.9, 171.9, 171.1, 117.3, 116.1, 78.4, 74.3, 37.6, 37.3, 37.2, 33.6, 28.9, 28.8, 28.1, 27.9, 25.7, 22.5, 21.1, 21.0, 20.3, 20.1, 9.4 ppm. GC/MS (EI): m/z (%): 194 (100) [*M*⁺], 179 (98), 165 (25), 151 (99), 139 (74), 125 (18), 55 (30).

### Odor description: green fruity, peppery, woody, guaïac, myraldyl.

*(2R,4R)*-2-ethyl-4-methyl-2,3,4,6,7,8-hexahydro-5H-chromen-5-one and *(2S,4R)*-2-ethyl-4-methyl-2,3,4,6,7,8-hexahydro-5H-chromen-5-one wrere isolated from *(4R)*-2-ethyl-4-methyl-2,3,4,6,7,8-hexahydro-5H-chromen-5-one via silica gel chromatography (Heptane: MTBE=20:1) as colorless liquid.

*(2R,4R)*-2-ethyl-4-methyl-2,3,4,6,7,8-hexahydro-5H-chromen-5-one: ¹H NMR (400 MHz, CDCl₃) δ 4.06 - 3.81 (m, 1H), 2.90 - 2.70 (m, 1H), 2.47 - 2.24 (m, 4H), 2.06 - 1.86 (m, 2H), 1.80 - 1.46 (m, 4H), 1.21 - 0.84 (m, 6H) ppm. ¹³C NMR (101 MHz, CDCl₃) δ = 197.9, 171.1, 116.1, 74.2, 37.2, 33.6, 28.8, 28.1, 22.5, 21.1, 21.0, 9.4 ppm. GC/MS (EI): m/z (%): 194 (100) [M⁺], 179 (97), 165 (24), 151 (99), 139 (74), 125 (18), 111 (18), 55 (31). Odor description: animalic, smokey, burnt, leathery, suede, styrax pyrogenee, cade

*(2S,4R)*-2-ethyl-4-methyl-2,3,4,6,7,8-hexahydro-5H-chromen-5-one: ¹H NMR (400 MHz, CDCl₃) δ 3.81 - 3.58 (m, 1H), 2.73 - 2.55 (m, 1H), 2.51 - 2.17 (m, 4H), 2.11 - 1.79 (m, 3H), 1.76 - 1.53 (m, 2H), 1.39 - 1.26 (m, 1H), 1.24 - 1.12 (m, 3H), 1.06 - 0.88 (m, 3H)ppm. ¹³C NMR (101 MHz, CDCl₃) δ = 197.9, 171.9, 117.3, 78.4, 37.6, 37.3, 28.9, 27.9, 25.7, 20.3, 20.1, 9.4 ppm. GC/MS (EI): m/z (%): 194 (67) [*M*⁺], 179 (42), 166 (12), 151 (60), 139 (100), 55 (22).

### Odor description: violet, woody, fatty fruity green

### Example 4-2: (4S)-2-ethyl-4-methyl-2,3,4,6,7,8-hexahydro-5H-chromen-5-one

1. According the procedure of example 4-1, the mixture of cyclohexane-1,3-dione (20g, 178mmol, 1.0 eq) and but-2-enal (14 g, 196mmol, 1.1 equiv) catalyzed by (*R*)-2-(diphenyl((trimethylsilyl)oxy)methyl)pyrrolidine (5.8 g, 18 mmol, 0.1 equiv) give *(4S)*-2-hydroxy-4-methyl-2,3,4,6,7,8-hexahydro-5H-chromen-5-one as white solid (84mmol, 15g, 47% yield).
   ¹H NMR (400 MHz, CDCl₃, mixture of diasteromers) δ 5.55 - 5.30 (m, 1H), 4.88 - 4.31 (m, 1H), 2.95 - 2.65 (m, 1H), 2.49 - 2.26 (m, 4H), 2.10 - 1.86 (m, 3H), 1.82 - 1.67 (m, 1H), 1.31 - 1.04 (m, 3H) ppm. ¹³C NMR (101 MHz, CDCl₃, mixture of diasteromers) δ 198.6, 198.4, 169.7, 168.8, 116.5, 115.9, 94.4, 93.1, 37.1, 37.0, 35.7, 35.6, 28.8, 28.6, 23.1, 22.8, 21.0, 20.9, 20.4, 20.3 ppm. GC/MS (EI): m/z (%): 182 (56) [*M*⁺], 167 (5), 153 (89), 139 (100), 126 (26), 111 (33), 55 (67).
2. The compound was obtained from (4S)-2-hydroxy-4-methyl-2,3,4,6,7,8-hexahydro-5H-chromen-5-one (10.0 g, 55 mmol) and ethylmagnesium bromide (110 ml, 110mmol, 1.0 M) according to the procedure of example 4-1 as colorless liquid (6.2 mmol, 1.2 g, 11% yield, trans:cis=1.8:1).

¹H NMR (400 MHz, CDCl₃, mixture of diasteromers) δ 4.06 - 3.50 (m, 1H), 2.91 - 2.48 (m, 1H), 2.45 - 2.15 (m, 4H), 2.04 - 1.26 (m, 6H), 1.21 - 1.05 (m, 3H), 1.05 - 0.93 (m, 3H)ppm. ¹³C NMR (101 MHz, CDCl₃, mixture of diasteromers) δ = 197.8, 171.8, 171.1, 117.2, 116.1, 78.4, 74.2, 37.6, 37.3, 37.2, 33.6, 28.9, 28.8, 28.1, 27.9, 25.7, 22.5, 21.1, 21.0, 20.3, 20.1, 9.5, 9.4 ppm. GC/MS (EI): m/z (%): 194 (86) [*M*⁺], 179 (86), 165 (23), 151 (100), 139 (85), 123 (20), 55 (49).

### Odor description: fruity, peppery, woody, myraldyl, isoraldéïne, guaïac

*(2S,4S)*-2-ethyl-4-methyl-2,3,4,6,7,8-hexahydro-5H-chromen-5-one was isolated from *(4S)*-2-ethyl-4-methyl-2,3,4,6,7,8-hexahydro-5H-chromen-5-one via silica gel chromatography (Heptane: MTBE=20:1) as colorless liquid.

¹H NMR (400 MHz, CDCl₃) δ 3.95 - 3.77 (m, 1H), 2.71 (dd, *J* = 9.3, 3.9 Hz, 1H), 2.41 - 2.19 (m, 4H), 1.86 (m, 2H), 1.70 - 1.41 (m, 4H), 1.07 - 0.87 (m, 6H) ppm. ¹³C NMR (101 MHz, CDCl₃) δ 198.0, 171.2, 116.2, 74.3, 37.2, 33.6, 28.8, 28.2, 22.6, 21.1, 21.0, 9.5 ppm. GC/MS (EI): m/z (%): 194 (100) [*M*⁺], 179 (95), 165 (25), 151 (99), 139 (73), 55 (30).

### Odor description: warm fruity woody,acet guayil, cedarwood, myraldyl, isoraldéïne

*(2R,4S)*-2-ethyl-4-methyl-2,3,4,6,7,8-hexahydro-5H-chromen-5-one was isolated from *(4S)*-2-ethyl-4-methyl-2,3,4,6,7,8-hexahydro-5H-chromen-5-one via silica gel chromatography (Heptane: MTBE=20:1) as colorless liquid.

¹H NMR (400 MHz, CDCl₃) δ 4.03 - 3.59 (m, 1H), 2.89 - 2.54 (m, 1H), 2.48 - 2.18 (m, 4H), 2.08 - 1.99 (m, 1H), 1.97 - 1.79 (m, 2H), 1.77 - 1.51 (m, 2H), 1.38 - 1.24 (m, 1H), 1.21 - 1.06 (m, 3H), 1.04 - 0.94 (m, 3H)ppm. ¹³C NMR (101 MHz, CDCl₃) δ 197.9, 171.9, 171.1, 117.3, 116.1, 78.4, 74.3, 37.6, 37.3, 37.2, 33.6, 28.9, 28.8, 28.1, 27.9, 25.7, 22.5, 21.1, 21.0, 20.3, 20.1, 9.4 ppm. GC/MS (EI): m/z (%): 194 (64) [M⁺], 179 (41), 166 (12), 151 (64), 139 (100), 55 (32).

### Odor description: fruity, woody, peppery.

### Example 4-3: (4R)-4-methyl-2-propyl-2,3,4,6,7,8-hexahydro-5H-chromen-5-one

The compound was obtained from (4R)-2-hydroxy-4-methyl-2,3,4,6,7,8-hexahydro-5H-chromen-5-one (5.0 g, 27mmol) and propylmagnesium bromide (27ml, 55 mmol, 2.0 M) according to the procedure of example 4-1 as colorless liquid (15 mmol, 3.1 g, 54% yield).

¹H NMR (400 MHz, CDCl₃, mixture of diasteromers) δ 4.08 - 3.50 (m, 1H), 2.80 - 2.45 (m, 1H), 2.42 - 2.09 (m, 4H), 2.04 - 1.17 (m, 8H), 1.17 - 0.96 (m, 3H), 0.95 - 0.79 (m, 3H) ppm. ¹³C NMR (101 MHz, CDCl₃, mixture of diasteromers) δ 198.0, 171.9, 171.1, 117.3, 116.2, 77.1, 72.9, 38.2, 37.4, 37.2, 37.1, 34.2, 29.0, 28.9, 25.8, 22.6, 21.1, 21.0, 20.4, 20.1, 18.4, 18.3, 14.0 ppm. GC/MS (EI): m/z (%): 208 (80) [*M*⁺], 193 (53), 179 (26), 151 (83), 139 (100), 125 (26), 109 (24), 55 (29).

### Odor description: peppery, green, woody, cedrenol, vetiver, grapefruit

*(2R,4R)-*4-methyl-2-propyl-2,3,4,6,7,8-hexahydro-5H-chromen-5-one was isolated from *(4R)-*4-methyl-2-propyl-2,3,4,6,7,8-hexahydro-5H-chromen-5-one via silica gel chromatography (Heptane: MTBE=20:1)
*(2R,4R)-isomer:* ¹H NMR (400 MHz, CDCl₃) δ 4.12 - 3.71 (m, 1H), 2.88 - 2.59 (m, 1H), 2.48 - 2.25 (m, 4H), 2.05 - 1.80 (m, 2H), 1.74 - 1.37 (m, 6H), 1.08 (d, *J* = 6.9 Hz, 3H), 1.01 - 0.88 (m, 3H) ppm. ¹³C NMR (101 MHz, CDCl₃) δ 197.9, 171.1, 116.1, 72.9, 37.3, 37.2, 34.1, 28.8, 22.6, 21.1, 21.0, 18.3, 14.0 ppm. GC/MS (EI): m/z (%): 208 (91) [*M*⁺], 193 (68), 179 (36), 151 (99), 139 (100), 125 (33), 55 (36).

### Odor description: peppery, green, woody

### Example 4-4: (4S)-4-methyl-2-propyl-2,3,4,6,7,8-hexahydro-5H-chromen-5-one

The compound was obtained from (4S)-2-hydroxy-4-methyl-2,3,4,6,7,8-hexahydro-5H-chromen-5-one (5.0 g, 27mmol) and propylmagnesium bromide (27ml, 55 mmol, 2.0 M) according to the procedure of example 4-1 as colorless liquid (14 mmol, 2.9 g, 51% yield, trans:cis=2.1:1).

*(2S,4S)*-4-methyl-2-propyl-2,3,4,6,7,8-hexahydro-5H-chromen-5-one: ¹H NMR (400 MHz, CDCl3) δ 4.11 - 3.91 (m, 1H), 2.88 - 2.69 (m, 1H), 2.46 - 2.27 (m, 4H), 2.04 - 1.84 (m, 2H), 1.74 - 1.38 (m, 6H), 1.15 - 1.03 (m, 3H), 1.02 - 0.90 (m, 3H)ppm. ¹³C NMR (101 MHz, CDCl₃) δ 197.9, 171.1, 116.1, 72.9, 37.3, 37.2, 34.1, 28.8, 22.6, 21.1, 21.0, 18.3, 14.0 ppm. GC/MS (EI): m/z (%): 208 (99) [*M*⁺], 193 (73), 179 (37), 151 (100), 139 (98), 125 (33), 55 (33). *(2R,4S)*-4-methyl-2-propyl-2,3,4,6,7,8-hexahydro-5H-chromen-5-one: ¹H NMR (400 MHz, CDCl₃) δ 3.99 - 3.58 (m, 1H), 2.82 - 2.48 (m, 1H), 2.39 - 2.10 (m, 4H), 2.02 - 1.70 (m, 3H), 1.66 - 1.19 (m, 5H), 1.13 - 0.99 (m, 3H), 0.93 - 0.82 (m, 3H) ppm. ¹³C NMR (101 MHz, CDCl₃) δ 198.0, 171.9, 171.1, 117.3, 116.1, 72.9, 38.2, 37.4, 37.2, 37.1, 34.1, 29.0, 28.8, 25.8, 22.6, 21.1, 21.0, 20.3, 20.1, 18.4, 18.3, 14.0 ppm. GC/MS (EI): m/z (%): 208 (55) [M⁺], 193 (32), 179 (12), 165 (12), 151 (63), 139 (100), (33).

### Odor description: fruity, green, acet vetikol, woody, cedarwood

### Example 4-5: (4R)-2-isopropyl-4-methyl-2,3,4,6,7,8-hexahydro-5H-chromen-5-one

The compound was obtained from (4R)-2-hydroxy-4-methyl-2,3,4,6,7,8-hexahydro-5H-chromen-5-one (3.0 g, 16mmol) and isopropylmagnesium chloride (16ml, 33mmol, 2.0 M) according to the procedure of example 4-1 as colorless liquid (14 mmol, 2.9 g, 84% yield, trans:cis=3:1).

¹H NMR (400 MHz, CDCl₃, mixture of diasteromers) δ 3.83 - 3.43 (m, 1H), 2.87 - 2.51 (m, 1H), 2.45 - 2.17 (m, 4H), 2.07 - 1.24 (m, 5H), 1.21 - 1.04 (m, 3H), 1.02 - 0.92 (m, 6H) ppm. ¹³C NMR (101 MHz, CDCl₃, mixture of diasteromers) δ 197.9, 172.2, 171.3, 117.2, 116.1, 81.9, 77.6, 37.3, 37.1, 34.8, 32.0, 31.8, 30.6, 28.9, 28.7, 26.0, 22.4, 21.0, 21.0, 20.3, 20.0, 18.1, 18.0, 17.8, 17.7 ppm. GC/MS (EI): m/z (%): 208 (59) [*M*⁺], 193 (29), 152 (30), 139 (100), 55 (22).

### Odor description: peppery, creamy woody, cedarwood, berry orris, fruity

### Example 4-6: (4S)-2-isopropyl-4-methyl-2,3,4,6,7,8-hexahydro-5H-chromen-5-one

The compound was obtained from (4S)-2-hydroxy-4-methyl-2,3,4,6,7,8-hexahydro-5H-chromen-5-one (6.0 g, 33 mmol, 1.0 equiv) and isopropylmagnesium bromide (66 ml, 66 mmol, 1.0 M) according to the procedure of example 4-1 as colorless liquid (8.3 mmol, 1.9 g, 27% yield, trans:cis=1:1).

¹H NMR (400 MHz, CDCl₃, mixture of diasteromers) δ 3.85 - 3.46 (m, 1H), 2.88 - 2.54 (m, 1H), 2.46 - 2.16 (m, 4H), 2.00 (m, 1H), 1.96 - 1.80 (m, 3H), 1.62 - 1.53 (m, 1H), 1.30 (m, 1H), 1.13 (dd, J = 41.4, 6.7 Hz, 3H), 1.03 - 0.94 (m, 6H) ppm. ¹³C NMR (101 MHz, CDCl₃, mixture of diasteromers) δ 198.0, 172.2, 171.4, 117.3, 116.2, 82.0, 77.7, 37.4, 37.2, 34.9, 32.1, 31.9, 30.6, 28.9, 28.8, 26.1, 22.5, 21.1, 21.0, 20.4, 20.1, 18.1, 18.0, 17.9, 17.8 ppm. GC/MS (EI): m/z (%): 208 (51) [*M*⁺], 193 (21), 165 (19), 152 (26), 139 (100), 55 (20).

### Odor description: woody, isoraldéïne, fruity, raspberry, sweet, peppery

### Example 4-7: (4R)-2-(sec-butyl)-4-methyl-2,3,4,6,7,8-hexahydro-5H-chromen-5-one

The compound was obtained from (4R)-2-hydroxy-4-methyl-2,3,4,6,7,8-hexahydro-5H-chromen-5-one (5.0 g, 27mmol) and sec-butylmagnesium bromide (41ml, 41mmol, 1.0 M) according to the procedure of example 4-1 as colorless liquid (5.4 mmol, 1.2 g, 20% yield).

¹H NMR (400 MHz, CDCl₃, mixture of isomers) δ 3.93 - 3.53 (m, 1H), 2.91 - 2.00 (m, 6H), 1.97 - 0.99 (m, 11H), 0.92 - 0.74 (m, 4H) ppm. ¹³C NMR (101 MHz, CDCl₃, mixture of isomers) δ = 198.0, 197.7, 171.5, 171.4, 169.0, 117.3, 116.3, 116.2, 116.1, 99.2, 98.4, 96.6, 96.2, 80.8, 80.6, 78.1, 76.5, 76.3, 75.8, 75.0, 38.5, 38.4, 38.3, 38.2, 37.3, 37.2, 37.1, 35.2, 35.1, 34.8, 34.2, 30.7, 29.9, 29.8, 29.3, 28.9, 28.7, 26.2, 26.0, 25.2, 25.1, 24.9, 22.9, 22.7, 22.6, 22.4, 21.1, 21.0, 20.9, 20.8, 20.7, 20.6, 20.3, 20.0, 19.2, 19.1, 14.2, 14.0, 11.6, 11.5, 11.4, 9.8, 9.7 ppm. GC/MS (EI): m/z (%): 222 (36) [*M*⁺], 207 (11), 193 (11), 179 (2), 165 (15), 151 (23), 139 (100), 55 (23).

### Odor description: fruity, creamy, woody, cedarwood, peppery

### Example 4-8: (4S)-2-(sec-butyl)-4-methyl-2,3,4,6,7,8-hexahydro-5H-chromen-5-one

The compound was obtained from (4S)-2-hydroxy-4-methyl-2,3,4,6,7,8-hexahydro-5H-chromen-5-one (6.0 g, 33mmol) and sec-butylmagnesium bromide (66ml, 66 mmol, 1.0 M) according to the procedure of example 4-1 as colorless liquid (16mmol, 3.5 g, 47% yield, trans:cis=2:1).

1H NMR (400 MHz, CDCl₃, mixture of isomers) δ 3.96 - 3.56 (m, 1H), 2.72 (m, 1H), 2.44 - 2.16 (m, 4H), 2.02 - 1.83 (m, 2H), 1.76 - 1.20 (m, 5H), 1.18 (d, *J* = 6.5 Hz, 1H), 1.08 (m, 2H), 1.00 - 0.85 (m, 6H) ppm. ¹³C NMR (101 MHz, CDCl₃, mixture of diasteromers) δ 197.9, 172.3, 172.2, 171.5, 171.4, 117.4, 116.2, 116.1, 80.8, 80.6, 76.5, 76.3, 38.6, 38.5, 38.4, 38.3, 37.4, 37.2, 35.2, 34.3, 30.8, 29.8, 28.9, 28.8, 28.7, 26.2, 26.1, 25.2, 25.1, 25.0, 24.9, 22.6, 22.5, 21.1, 21.0, 20.4, 20.3, 20.1, 14.3, 14.2, 14.1, 14.0, 11.6, 11.5, 11.5, 11.5 ppm. GC/MS (EI): m/z (%): 222 (45) [*M*⁺], 207 (13), 193 (12), 139 (100), 55 (21).

### Odor description: woody, acet guayïl, rotundone, peppery fatty fruity, isoraldéïne

Example 4-9: *(4R)*-4-ethyl-2-methyl-2,3,4,6,7,8-hexahydro-5H-chromen-5-one 1.*(4R)*-4-ethyl-2-hydroxy-2,3,4,6,7,8-hexahydro-5H-chromen-5-one was obtained from cyclohexane-1,3-dione (50g, 446 mmol, 1.0 equiv) and pent-2-enal (41g, 491 mmol, 1.1 equiv) catalyzed by (S)-2-(diphenyl((trimethylsilyl)oxy)methyl)pyrrolidine (14.5 g, 44.6 mmol, 0.1 equiv) according to the procedure of example 4-1 as white solid (294 mmol, 59 g, 66% yield).

¹H NMR (400 MHz, CDCl₃, mixture of diasteromers) δ 5.67 - 5.01 (m, 2H), 2.75 - 2.54 (m, 1H), 2.49 - 2.23 (m, 4H), 2.15 - 1.41 (m, 5H), 1.30 - 1.05 (m, 1H), 1.01 - 0.73 (m, 3H)ppm. ¹³C NMR (101 MHz, CDCl₃, mixture of diasteromers) δ 198.6, 170.3, 169.9, 115.8, 115.2, 94.8, 93.3, 37.1, 36.9, 31.7, 31.3, 29.7, 29.3, 28.9, 28.7, 26.9, 25.6, 20.9, 20.3, 11.6, 11.0 ppm. GC/MS (EI): m/z (%): 196 (38) [*M*⁺], 178 (8), 167 (86), 153 (59), 139 (100), 125 (33), 79 (30), 55 (47).

2. The compound was obtained from (4R)-4-ethyl-2-hydroxy-2,3,4,6,7,8-hexahydro-5H-chromen-5-one (5.0 g, 26 mmol) and methylmagnesium bromide (13ml, 38mmol, 3.0 M) according to the procedure of example 4-1 as colorless liquid (5.2 mmol, 1.0 g, 20% yield).

¹H NMR (400 MHz, CDCl₃, mixture of diasteromers) δ 4.23 - 3.77 (m, 1H), 2.65 - 2.47 (m, 1H), 2.46 - 2.14 (m, 4H), 2.09 - 1.60 (m, 4H), 1.47 - 1.02 (m, 5H), 0.98 - 0.73 (m, 3H)ppm. ¹³C NMR (101 MHz, CDCl₃, mixture of diasteromers) δ 197.9, 197.8, 172.5, 170.9, 116.0, 115.4, 73.5, 69.5, 37.3, 37.2, 36.3, 31.5, 31.4, 29.4, 29.0, 28.9, 27.0, 26.0, 21.1, 21.0, 20.9, 20.0, 11.7, 10.3 ppm. GC/MS (EI): m/z (%): 194 (35) [M⁺], 179 (9), 165 (100), 151 (6), 137 (23), 55 (13).

### Odor description: woody, cedarwood, powdery, green

### Example 4-10: (4S)-4-ethyl-2-methyl-2,3,4,6,7,8-hexahydro-5H-chromen-5-one

1. *(4S)*-4-ethyl-2-hydroxy-2,3,4,6,7,8-hexahydro-5H-chromen-5-one was obtained from cyclohexane-1,3-dione (10.0 g, 89mmol, 1.0 equiv) and pent-2-enal (8.3 g, 98mmol, 1.1 equiv) catalyzed by (R)-2-(diphenyl((trimethylsilyl)oxy)methyl)pyrrolidine (3.2g, 45 mmol, 0.1 equiv) according to the procedure of example 4-1 as white solid (68 mmol, 13.3 g, 76% yield).
   ¹H NMR (400 MHz, CDCl₃, mixture of diasteromers) δ 5.45 - 5.32 (m, 1H), 4.97 - 4.30 (m, 1H), 2.70 - 2.54 (m, 1H), 2.49 - 2.25 (m, 4H), 2.16 - 1.41 (m, 5H), 1.33 - 1.06 (m, 1H), 1.00 - 0.83 (m, 3H) ppm. ¹³C NMR (101 MHz, CDCl₃, mixture of diasteromers) δ 198.3, 169.8, 169.8, 169.4, 115.9, 115.3, 94.7, 93.2, 37.1, 37.0, 31.5, 31.3, 29.7, 29.2, 28.9, 28.7, 26.9, 25.7, 20.9, 20.3, 11.6, 11.1 ppm. GC/MS (EI): m/z (%): 196 (53) [*M*⁺], 178 (10), 167 (99), 153 (66), 139 (100), 125 (31), 107 (24), 97 (20), 79 (26), 69 (16), 55 (45).
2. (4S)-4-ethyl-2-methyl-2,3,4,6,7,8-hexahydro-5H-chromen-5-one was obtained from (4S)-4-ethyl-2-hydroxy-2,3,4,6,7,8-hexahydro-5H-chromen-5-one (6.0 g, 31 mmol) and methylmagnesium bromide (20ml, 61mmol, 3.0 M) according to the procedure of example 4-1 as colorless liquid (13mmol, 2.5 g, 43% yield).

¹H NMR (400 MHz, CDCl₃, mixture of diasteromers) δ 4.22 - 3.78 (m, 1H), 2.65 - 2.46 (m, 1H), 2.45 - 2.17 (m, 4H), 2.13 - 1.59 (m, 4H), 1.49 - 1.03 (m, 5H), 0.99 - 0.71 (m, 3H)ppm. ¹³C NMR (101 MHz, CDCl₃, mixture of diasteromers) δ 197.9, 197.8, 172.6, 171.0, 116.1, 115.7, 77.1, 73.1, 37.5, 37.4, 37.3, 37.2, 34.5, 31.4, 29.6, 29.3, 29.1, 28.9, 27.0, 26.1, 21.1, 20.1, 18.4, 18.3, 14.0, 11.7, 10.3 ppm. GC/MS (EI): m/z (%): 194 (35) [M⁺], 179 (9), 165 (100), 151 (6), 137 (21), 55 (10).

### Odor description: woody, green, peppery

### Example 4-11: (4R)-2,4-diethyl-2,3,4,6,7,8-hexahydro-5H-chromen-5-one

The title compound was obtained from (4R)-4-ethyl-2-hydroxy-2,3,4,6,7,8-hexahydro-5H-chromen-5-one (5.0 g, 26 mmol) and ethylmagnesium bromide (51ml, 51mmol, 1.0 M) according to the procedure of example 4-1 as colorless liquid (9.1 mmol, 1.9 g, 36% yield, trans:cis=1.8:1).

¹H NMR (400 MHz, CDCl₃, mixture of diasteromers) δ 4.00 - 3.54 (m, 1H), 2.63 - 2.47 (m, 1H), 2.45 - 2.16 (m, 4H), 2.10 - 1.19 (m, 8H), 1.13 - 0.96 (m, 3H), 0.95 - 0.77 (m, 3H) ppm.¹³C NMR (101 MHz, CDCl₃, mixture of diasteromers) δ = 198.0, 197.9, 172.6, 171.0, 116.1, 115.7, 78.5, 74.4, 37.4, 37.2, 33.9, 31.4, 29.3, 29.1, 29.0, 28.9, 28.2, 28.0, 27.0, 26.0, 21.0, 20.0, 11.7, 10.3, 9.5, 9.4 ppm. GC/MS (EI): m/z (%): 208 (31) [*M*⁺], 193 (10), 179 (100), 165 (8), 153 (18), 137 (27), 55 (27).

### Odor description: woody, cedarwood, green, peppery

*(2R,4R)*-2,4-diethyl-2,3,4,6,7,8-hexahydro-5H-chromen-5-one was isolated from *(4R)-*2,4-diethyl-2,3,4,6,7,8-hexahydro-5H-chromen-5-one via silica gel chromatography (Heptane: MTBE=20:1) as colorless liquid.

¹H NMR (400 MHz, CDCl₃) δ 3.98 - 3.82 (m, 1H), 2.59 - 2.48 (m, 1H), 2.44 - 2.25 (m, 4H), 1.99 - 1.89 (m, 2H), 1.87 - 1.79 (m, 1H), 1.77 - 1.55 (m, 3H), 1.45 - 1.33 (m, 1H), 1.16 - 1.05 (m, 1H), 1.04 - 0.97 (m, 3H), 0.92 (t, *J* = 7.4 Hz, 3H) ppm. ¹³C NMR (101 MHz, CDCl₃) δ 197.8, 171.0, 115.6, 74.4, 37.2, 29.2, 29.0, 28.8, 28.2, 27.0, 21.0, 11.7, 9.4 ppm. GC/MS (EI): m/z (%): 208 (38) [*M*⁺], 193 (10), 179 (100), 137 (18), 125 (21), 55 (12).

### Odor description: woody, cedarwood, peppery

### Example 4-12: (4S)-2,4-diethyl-2,3,4,6,7,8-hexahydro-5H-chromen-5-one

The title compound was obtained from (*4S*)-4-ethyl-2-hydroxy-2,3,4,6,7,8-hexahydro-5H-chromen-5-one (5.0 g, 26 mmol) and ethylmagnesium bromide (51ml, 51mmol, 1.0 M) according to the procedure of example 4-1 as colorless liquid (16mmol, 3.4 g, 64% yield, trans:cis=1.7:1).

¹H NMR (400 MHz, CDCl₃, mixture of diasteromers) δ 3.99 - 3.57 (m, 1H), 2.64 - 2.45 (m, 1H), 2.45 - 2.18 (m, 4H), 2.15 - 1.55 (m, 6H), 1.50 - 0.96 (m, 5H), 0.95 - 0.74 (m, 3H) ppm. ¹³C NMR (101 MHz, CDCl₃, mixture of diasteromers) δ = 197.9, 197.8, 172.6, 171.0, 116.0, 115.6, 78.4, 74.4, 37.3, 37.1, 33.9, 31.3, 29.2, 29.1, 29.0, 28.8, 28.2, 27.9, 26.9, 26.0, 21.1, 20.0, 11.6, 10.3, 9.5, 9.4 ppm. GC/MS (EI): m/z (%): 208 (42) [*M*⁺], 193 (10), 179 (100), 153 (20), 137 (21), 125 (23), 55 (13).

### Odor description: woody, peppery, green, fruity

*(2S,4S)*-2,4-diethyl-2,3,4,6,7,8-hexahydro-5H-chromen-5-one was isolated from (4S)-2,4-diethyl-2,3,4,6,7,8-hexahydro-5H-chromen-5-one via silica gel chromatography (Heptane: MTBE=20:1) as colorless liquid.

¹H NMR (400 MHz, CDCl₃) δ 3.99 - 3.82 (m, 1H), 2.62 - 2.47 (m, 1H), 2.45 - 2.26 (m, 4H), 2.01 - 1.80 (m, 3H), 1.78 - 1.55 (m, 3H), 1.48 - 1.32 (m, 1H), 1.17 - 1.05 (m, 1H), 1.04 - 0.96 (m, 3H), 0.95 - 0.86 (m, 3H) ppm. ¹³C NMR (101 MHz, CDCl₃) δ 197.8, 171.0, 115.6, 74.4, 37.2, 29.2, 29.0, 28.8, 28.2, 26.9, 21.0, 11.6, 9.4 ppm. GC/MS (EI): m/z (%): 208 (35) [M⁺], 193 (10), 179 (100), 151 (11), 137 (18), 55 (13).

### Odor description: woody, peppery, green.

### Example 4-13: (4R)-4-ethyl-2-propyl-2,3,4,6,7,8-hexahydro-5H-chromen-5-one

The compound was obtained from (4R)-4-ethyl-2-hydroxy-2,3,4,6,7,8-hexahydro-5H-chromen-5-one (5.0 g, 26 mmol) and propylmagnesium bromide (26 ml, 51 mmol, 2.0 M) according to the procedure of example 4-1 as colorless liquid (17 mmol, 3.7 g, 65% yield, trans:cis=2.5:1).

¹H NMR (400 MHz, CDCl₃, mixture of diasteromers) δ 4.08 - 3.58 (m, 1H), 2.65 - 2.48 (m, 1H), 2.46 - 2.16 (m, 4H), 2.10 - 1.81 (m, 3H), 1.76 - 1.28 (m, 6H), 1.22 - 0.55 (m, 7H) ppm. ¹³C NMR (101 MHz, CDCl₃, mixture of diasteromers) δ 197.9, 197.8, 172.6, 171.0, 116.1, 115.7, 77.1, 73.1, 37.5, 37.4, 37.3, 37.2, 34.5, 31.4, 29.6, 29.3, 29.1, 28.9, 27.0, 26.1, 21.1, 20.1, 18.4, 18.3, 14.0, 11.7, 10.3 ppm. GC/MS (EI): m/z (%): 222 (37) [*M*⁺], 207 (7), 193 (100), 179 (3), 165 (10), 153 (15), 137 (15), 125 (40), 55 (15).

### Odor description: woody, peppery, guayil, green

### Example 4-14: (4S)-4-ethyl-2-propyl-2,3,4,6,7,8-hexahydro-5H-chromen-5-one

The compound was obtained from (4S)-4-ethyl-2-hydroxy-2,3,4,6,7,8-hexahydro-5H-chromen-5-one (3.5 g, 18 mmol) and propylmagnesium bromide (18 ml, 36 mmol, 2.0 M) according to the procedure of example 4-1 as colorless liquid (13mmol, 2.9 g, 73% yield, trans:cis=2.1:1).

¹H NMR (400 MHz, CDCl₃, mixture of diasteromers) δ 4.09 - 3.58 (m, 1H), 2.64 - 2.47 (m, 1H), 2.46 - 2.16 (m, 4H), 2.05 - 1.24 (m, 9H), 1.19 - 0.70 (m, 7H) ppm.¹³C NMR (101 MHz, CDCl₃, mixture of diasteromers) δ 198.0, 197.9, 172.7, 171.1, 116.1, 115.7, 77.1, 73.1, 37.5, 37.4, 37.3, 34.5, 31.4, 29.6, 29.3, 29.1, 28.9, 27.0, 26.1, 21.1, 20.0, 18.4, 18.3, 14.0, 11.7, 10.4 ppm. GC/MS (EI): m/z (%): 222 (38) [*M*⁺], 207 (7), 193 (100), 165 (11), 153 (18), 125 (40), 55 (14).

### Odor description: green, woody,vetiver root, cedrenol, peppery

### Example 4-15: (4R)-4-ethyl-2-isopropyl-2,3,4,6,7,8-hexahydro-5H-chromen-5-one

The compound was obtained from (4R)-4-ethyl-2-hydroxy-2,3,4,6,7,8-hexahydro-5H-chromen-5-one (4.0 g, 20.4 mmol, 1.0 equiv) and isopropylmagnesium bromide (20ml, 41 mmol, 2.0 M) according to the procedure of example 4-1 as colorless liquid (13mmol, 2.8 g, 62% yield, trans:cis=3.2:1).

¹H NMR (400 MHz, CDCl₃, mixture of diasteromers) δ 3.94 - 3.31 (m, 1H), 2.63 - 2.48 (m, 1H), 2.45 - 2.17 (m, 4H), 2.10 - 1.61 (m, 5H), 1.51 - 1.05 (m, 2H), 1.02 - 0.76 (m, 9H) ppm. ¹³C NMR (101 MHz, CDCl₃, mixture of diasteromers) δ = 197.9, 197.8, 172.9, 171.2, 116.1, 115.7, 81.9, 77.7, 37.4, 37.2, 32.1, 32.0, 31.6, 31.1, 29.1, 29.0, 28.8, 26.9, 26.0, 25.9, 21.1, 20.0, 18.1, 17.9, 17.8, 11.6, 10.3. GC/MS (EI): m/z (%): 222 (57) [M⁺], 207 (14), 193 (100), 153 (40), 137 (47), 125 (30), 55 (20).

Odor description: woody, green rooty vetiver, slightly ambery powdery, orris isoraldéïne, peppery

### Example 4-16: (4S)-4-ethyl-2-isopropyl-2,3,4,6,7,8-hexahydro-5H-chromen-5-one

The compound was obtained from (4S)-4-ethyl-2-hydroxy-2,3,4,6,7,8-hexahydro-5H-chromen-5-one (3.0 g, 15 mmol) and isopropylmagnesium bromide (16ml, 31 mmol, 2.0 M) according to the procedure of example 4-1 as colorless liquid (6.8 mmol, 1.5 g, 44% yield, trans:cis=6:1).

¹H NMR (400 MHz, CDCl₃, mixture of diasteromers) δ 4.00 - 3.43 (m, 1H), 2.67 - 2.16 (m, 5H), 2.11 - 1.47 (m, 5H), 1.46 - 1.05 (m, 2H), 1.03 - 0.77 (m, 9H) ppm. ¹³C NMR (101 MHz, CDCl₃, mixture of diasteromers) δ 197.9, 197.8, 172.9, 171.2, 116.0, 115.7, 115.5, 81.9, 77.7, 37.3, 37.2, 32.0, 31.9, 31.6, 29.1, 29.0, 28.8, 26.9, 26.0, 25.9, 21.0, 20.0, 18.1, 17.8, 17.7, 11.6, 11.5, 10.3 ppm. GC/MS (EI): m/z (%): 222 (54) [*M*⁺], 207 (15), 193 (100), 153 (32), 137 (47), 125 (31), 55 (23).

### Odor description: violet, woody, peppery, fruity

### Example 4-17: (4R)-2-(sec-butyl)-4-ethyl-2,3,4,6,7,8-hexahydro-5H-chromen-5-one

The compound was obtained from (4R)-4-ethyl-2-hydroxy-2,3,4,6,7,8-hexahydro-5H-chromen-5-one (5.0 g, 25mmol) and sec-butylmagnesium bromide (51ml, 51mmol, 1.0 M) according to the procedure of example 4-1 as colorless liquid (18 mmol, 4.2 g, 70% yield, trans: cis=2.1:1).

¹H NMR (400 MHz, CDCl₃, mixture of isomers) δ 3.94 - 3.48 (m, 1H), 2.62 - 2.47 (m, 1H), 2.45 - 2.14 (m, 4H), 2.06 - 1.52 (m, 6H), 1.49 - 1.04 (m, 3H), 1.01 - 0.77 (m, 9H) ppm. ¹³C NMR (101 MHz, CDCl₃, mixture of isomers) δ = 197.8, 197.7, 173.0, 172.9, 171.3, 171.2, 116.1, 115.8, 115.7, 80.8, 80.5, 38.6, 38.5, 38.4, 38.3, 37.3, 37.2, 31.8, 31.6, 31.4, 30.4, 29.3, 29.1, 29.0, 28.9, 28.8, 26.9, 26.1, 26.0, 25.2, 25.1, 25.0, 24.9, 21.1, 20.0, 14.3, 14.2, 14.1, 14.0, 11.9, 11.8, 11.7, 11.6, 11.5, 11.4, 10.4, 10.3 ppm. GC/MS (EI): m/z (%): 236 (48) [*M*⁺], 221 (7), 207 (100), 153 (45), 137 (27), 125 (31), 55 (19).

### Odor description: green, citrus, grapefruit, green, woody, peppery

### Example 4-18: (4S)-2-(sec-butyl)-4-ethyl-2,3,4,6,7,8-hexahydro-5H-chromen-5-one

The compound was obtained from (4S)-4-ethyl-2-hydroxy-2,3,4,6,7,8-hexahydro-5H-chromen-5-one (5.0 g, 2mmol) and sec-butylmagnesium bromide (51ml, 51mmol, 1.0 M) according to the procedure of example 4-1 as colorless liquid (18mmol, 4.3 g, 71% yield, trans:cis=2:1).

¹H NMR (400 MHz, CDCl₃, mixture of isomers) δ 3.96 - 3.53 (m, 1H), 2.60 - 2.49 (m, 1H), 2.45 - 2.17 (m, 4H), 2.05 - 1.88 (m, 2H), 1.82 - 1.65 (m, 2H), 1.64 - 1.03 (m, 5H), 0.99 - 0.78 (m, 9H) ppm. ¹³C NMR (101 MHz, CDCl₃, mixture of isomers) δ 197.9, 197.8, 173.1, 173.0, 171.4, 171.3, 116.2, 115.8, 115.8, 80.9, 80.6, 76.6, 76.5, 38.7, 38.6, 38.5, 38.4, 37.4, 37.3, 31.9, 31.7, 31.5, 30.5, 29.4, 29.2, 29.1, 28.9, 28.8, 26.9, 26.1, 26.0, 25.2, 25.1, 25.0, 24.9, 21.1, 20.1, 14.4, 14.3, 14.2, 14.1, 11.7, 11.7, 11.6, 11.6, 11.5, 11.5, 10.4 ppm. GC/MS (EI): m/z (%): 236 (48) [M⁺], 221 (6), 207 (100), 189 (4), 153 (51), 137 (29), 125 (32), 55 (21).

### Odor description: fruity woody, peppery green, grapefruit

### Example 5: Fine Fragrance accord

| Ingredient | parts by weight |
|---|---|
| AMBROFIX (3a,6,6,9a-tetramethyldodecahydronaphtho[2,1-b]furan) | 40 |
| CASHMERAN (6,7-dihydro-1,1,2,3,3-pentamethyl-4(5h)-indanone) | 40 |
| CEPIONATE (methyl 3-oxo-2-pentylcyclopentaneacetate) | 80 |
| COUMARIN PURE CRYSTALS (2h-1-benzopyran-2-one) | 10 |
| ETHYL LINALOOL (3,7-dimethyl-1,6-nonadien-3-ol) | 60 |
| ETHYL LINALYL ACETATE (3,7-dimethyl-1,6-nonadien-3-yl acetate) | 30 |
| ETHYL MALTOL @1wt% in benzyl alcohol (3-hydroxy-2-ethyl-4h-pyran-4-one) | 10 |
| HEXENYL-3-CIS SALICYLATE FINE (cis-3-hexenyl 2-hydroxybenzoate) | 60 |
| ISO E SUPER (2-acetyl-1,2,3,4,5,6,7,8-octahydro-2,3,8,8-tetra-methylnaphtalene) | 40 |
| MEFROSOL (3-methyl-5-phenyl-1-pentanol) | 20 |
| METHYL ANTHRANILATE EXTRA (methyl 2-aminobenzoate) | 5 |
| NYMPHEAL (3-(4-isobutyl-2-methylphenyl)propanal) | 5 |
| PETALIA (cyclohexylidene-o-tolyl-acetonitrile) | 10 |
| THIBETOLIDE (cyclopentadecanolide) | 60 |
| TROPIONAL (3-(3,4-methylenedioxyphenyl)-2-methylpropanal) | 20 |
| VANILLIN | 10 |
| Dipropylen Glycol (DPG) | 500 |
| TOTAL | 1000 |

The fragrance accord above is a powdery floral oriental accord, e.g., suitable to be applied @ 10 wt% to ethanol (85% vol).

By replacement 10 parts DPG with 10 parts of a mixture comprising 2,4-diethyl-2,3,4,6,7,8-hexahydro-5H-chromen-5-one and 2-ethyl-4-methyl-2,3,4,6,7,8-hexahydro-5H-chromen-5-one (Example 2-1) results in an accord with a powdery ambery facet, with a very natural soft peppery and powdery cedarwood impression. The addition brings a noticeable increase in the longlastingness, which clearly benefits the overall performance of the accord.

By replacement 10 parts DPG with 10 parts of a mixture comprising 4-methyl-2-propyl-2,3,4,6,7,8-hexahydro-5H-chromen-5-one and 2-ethyl-4-methyl-2,3,4,6,7,8-hexahydro-5H-chromen-5-one (Example 1-38) results in an accord with round creamy facet, with a very natural peppery and sandalwood impression. The addition brings a noticeable increase in the longlastingness, which clearly benefits the overall performance of the accord.

By replacing 10 parts DPG with 10 parts 2-ethyl-4-methyl-2,3,4,6,7,8-hexahydro-5H-chromen-5-one (Example 1-1) results in an accord with a fruity musky blackberry facet, with a very natural peppery and creamy sandalwood impression. And again, the addition brings a noticeable increase in the longlastingness, which clearly benfits the overall performance of the accord. Furthermore, the additon of 2-ethyl-4-methyl-2,3,4,6,7,8-hexahydro-5H-chromen-5-one enhances the peppery, woody and Rotundone facet.

## Claims

1. A compound of formula (I) 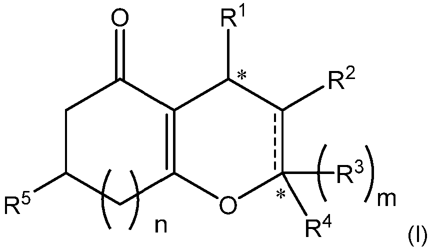 wherein
R¹ is selected from C₁ - C₅ alkyl,
R² is selected from hydrogen, C₁-C₃ alkyl,
R³ is selected from hydrogen, methyl and ethyl,
R⁴ is selected from C₁ - C₆ alkyl, C₂-C₆ alkenyl, C₃-C₆ cycloalkyl, and C₅-C₆ cycloalkenyl,
R⁵ is selected from hydrogen and methyl,
n is 0 or 1, and
the dotted line represents together with the C-C bond a single bond and m is 1, or the dotted line represents together with the C-C bond a double bond and m is 0.

2. A compound according to claim 1 wherein the compound of formula (I) is a compound of formula (II) 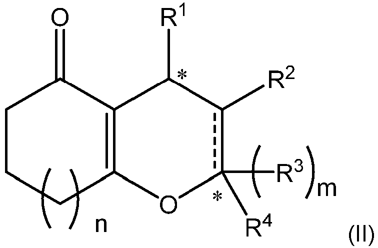 wherein
R¹ is selected from C₁ - C₅ alkyl,
R² is selected from hydrogen, C₁-C₃ alkyl,
R³ is selected from hydrogen, methyl and ethyl,
R⁴ is selected from C₁ - C₆ alkyl, C₂-C₆ alkenyl, C₃-C₆ cycloalkyl, and C₅-C₆ cycloalkenyl,
n is 0 or 1, and
the dotted line represents together with the C-C bond a single bond and m is 1, or the dotted line represents together with the C-C bond a double bond and m is 0.

3. A compound according to claim 1 wherein the compound of formula (I) is a compound of formula (III) 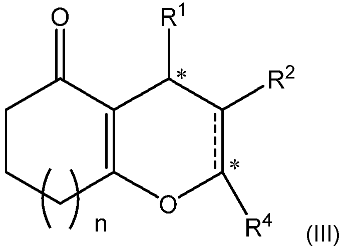 wherein
R¹ is selected from C₁ - C₅ alkyl,
R² is selected from hydrogen, C₁-C₃ alkyl,
R⁴ is selected from C₁ - C₆ alkyl, C₂-C₆ alkenyl, C₃-C₆ cycloalkyl, and C₅-C₆ cycloalkenyl,
n is 0 or 1, and
the dotted line represents together with the C-C bond a single bond or a double bond.

4. A compound according to claim 1 selected from the group consisting of 2-(but-2-en-2-yl)-4-ethyl-2,3,4,6,7,8-hexahydro-5H-chromen-5-one; 2-(but-2-en-2-yl)-4-methyl-2,3,4,6,7,8-hexahydro-5H-chromen-5-one; 2-(but-3-en-2-yl)-4-ethyl-2,3,4,6,7,8-hexahydro-5H-chromen-5-one; 2-(but-3-en-2-yl)-4-methyl-2,3,4,6,7,8-hexahydro-5H-chromen-5-one; 2-(sec-butyl)-4-ethyl-2,3,4,6,7,8-hexahydro-5H-chromen-5-one; 2-(sec-butyl)-4-methyl-2,3,4,6,7,8-hexahydro-5H-chromen-5-one; 2-(tert-butyl)-4-ethyl-2,3,4,6,7,8-hexahydro-5H-chromen-5-one; 2,2,4-trimethyl-2,3,4,6,7,8-hexahydro-5H-chromen-5-one; 2,3,4-trimethyl-4,6,7,8-tetrahydro-5H-chromen-5-one; 2,4-diethyl-2,3,4,6,7,8-hexahydro-5H-chromen-5-one; 2,4-diethyl-3,4,6,7-tetrahydrocyclopenta[b]pyran-5(2H)-one; 2,4-diethyl-3-methyl-2,3,4,6,7,8-hexahydro-5H-chromen-5-one; 2,4-diethyl-4,6,7,8-tetrahydro-5H-chromen-5-one; 2,4-dimethyl-2,3,4,6,7,8-hexahydro-5H-chromen-5-one; 2,4-dimethyl-2,3,4,6,7,8-hexahydro-5H-chromen-5-one; 2,4-dimethyl-4,6,7,8-tetrahydro-5H-chromen-5-one; 2-allyl-4-ethyl-2,3,4,6,7,8-hexahydro-5H-chromen-5-one; 2-allyl-4-methyl-2,3,4,6,7,8-hexahydro-5H-chromen-5-one; 2-butyl-4-ethyl-2,3,4,6,7,8-hexahydro-5H-chromen-5-one; 2-butyl-4-methyl-2,3,4,6,7,8-hexahydro-5H-chromen-5-one; 2-cyclopropyl-4-ethyl-2,3,4,6,7,8-hexahydro-5H-chromen-5-one; 2-cyclopropyl-4-methyl-2,3,4,6,7,8-hexahydro-5H-chromen-5-one; 2-ethyl-3,4-dimethyl-2,3,4,6,7,8-hexahydro-5H-chromen-5-one; 2-ethyl-4-(prop-1-en-1-yl)-2,3,4,6,7,8-hexahydro-5H-chromen-5-one; 2-ethyl-4,7-dimethyl-2,3,4,6,7,8-hexahydro-5H-chromen-5-one; 2-ethyl-4-methyl-2,3,4,6,7,8-hexahydro-5H-chromen-5-one; 2-ethyl-4-propyl-2,3,4,6,7,8-hexahydro-5H-chromen-5-one; 2-isobutyl-4-methyl-2,3,4,6,7,8-hexahydro-5H-chromen-5-one; 2-isopropyl-4-methyl-2,3,4,6,7,8-hexahydro-5H-chromen-5-one; 2-isopropyl-4-methyl-2,3,4,6,7,8-hexahydro-5H-chromen-5-one; 4-ethyl-2-(2-methylallyl)-2,3,4,6,7,8-hexahydro-5H-chromen-5-one; 4-ethyl-2-(2-methylprop-1-en-1-yl)-2,3,4,6,7,8-hexahydro-5H-chromen-5-one; 4-ethyl-2-(prop-1-en-1-yl)-2,3,4,6,7,8-hexahydro-5H-chromen-5-one; 4-ethyl-2-(prop-1-en-2-yl)-2,3,4,6,7,8-hexahydro-5H-chromen-5-one; 4-ethyl-2,3-dimethyl-2,3,4,6,7,8-hexahydro-5H-chromen-5-one; 4-ethyl-2-isobutyl-2,3,4,6,7,8-hexahydro-5H-chromen-5-one; 4-ethyl-2-isopropyl-2,3,4,6,7,8-hexahydro-5H-chromen-5-one; 4-ethyl-2-methyl-2,3,4,6,7,8-hexahydro-5H-chromen-5-one; 4-ethyl-2-propyl-2,3,4,6,7,8-hexahydro-5H-chromen-5-one; 4-ethyl-2-propyl-3,4,6,7-tetrahydrocyclopenta[b]pyran-5(2H)-one; 4-ethyl-2-vinyl-2,3,4,6,7,8-hexahydro-5H-chromen-5-one; 4-isopropyl-2-methyl-2,3,4,6,7,8-hexahydro-5H-chromen-5-one; 4-methyl-2-(2-methylprop-1-en-1-yl)-2,3,4,6,7,8-hexahydro-5H-chromen-5-one; 4-methyl-2-(prop-1-en-2-yl)-2,3,4,6,7,8-hexahydro-5H-chromen-5-one; and 4-methyl-2-propyl-2,3,4,6,7,8-hexahydro-5H-chromen-5-one; and mixtures thereof.

5. A fragrance composition comprising a compound of formula (I) as defined in any of claims 1 to 4, or a mixture thereof.

6. A fragranced article comprising a compound of formula (I) as defined in any of claims 1 to 4, or a mixture thereof, and a consumer product base.

7. A fragranced article according to claim 6 wherein the consumer product base is selected from fine perfumery, personal care products and fabric care products.

8. The use as fragrance of a compound of formula (I) as defined in any of claims 1 to 4, or a mixture thereof.

9. A method of improving, enhancing and/or modifying a consumer product base by means of adding thereto an olfactory acceptable amount of a compound of formula (I) as defined in any of claims 1 to 4, or a mixture thereof.

## Patentansprüche

1. Verbindung der Formel (I) 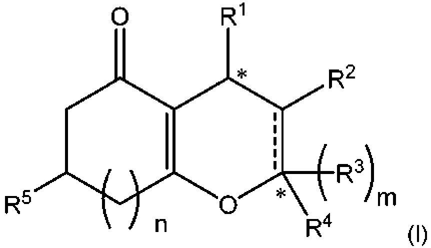 wobei
R¹ aus C₁-C₅-Alkyl ausgewählt ist,
R² aus Wasserstoff, C₁-C₃-Alkyl ausgewählt ist,
R³ aus Wasserstoff, Methyl und Ethyl ausgewählt ist,
R⁴ aus C₁-C₆-Alkyl, C₂-C₆-Alkenyl, C₃-C₆-Cycloalkyl und C₅-C₆-Cycloalkenyl ausgewählt ist,
R⁵ aus Wasserstoff und Methyl ausgewählt ist,
n für 0 oder 1 steht und
die gestrichelte Linie zusammen mit der C-C-Bindung eine Einfachbindung darstellt und m für 1 steht oder
die gestrichelte Linie zusammen mit der C-C-Bindung eine Doppelbindung darstellt und m für 0 steht.

2. Verbindung nach Anspruch 1, wobei es sich bei der Verbindung der Formel (I) um eine Verbindung der Formel (II) 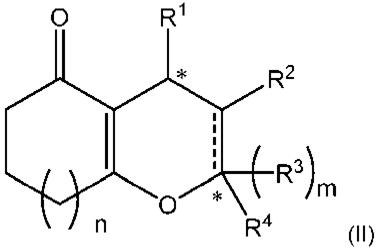 handelt, wobei
R¹ aus C₁-C₅-Alkyl ausgewählt ist,
R² aus Wasserstoff, C₁-C₃-Alkyl ausgewählt ist,
R³ aus Wasserstoff, Methyl und Ethyl ausgewählt ist,
R⁴ aus C₁-C₆-Alkyl, C₂-C₆-Alkenyl, C₃-C₆-Cycloalkyl und C₅-C₆-Cycloalkenyl ausgewählt ist,
n für 0 oder 1 steht und
die gestrichelte Linie zusammen mit der C-C-Bindung eine Einfachbindung darstellt und m für 1 steht oder
die gestrichelte Linie zusammen mit der C-C-Bindung eine Doppelbindung darstellt und m für 0 steht.

3. Verbindung nach Anspruch 1, wobei es sich bei der Verbindung der Formel (I) um eine Verbindung der Formel (III) 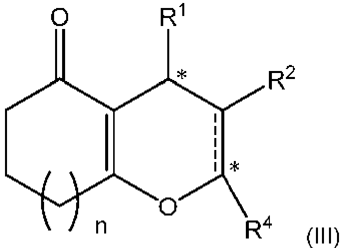 handelt, wobei
R¹ aus C₁-C₅-Alkyl ausgewählt ist,
R² aus Wasserstoff, C₁-C₃-Alkyl ausgewählt ist,
R⁴ aus C₁-C₆-Alkyl, C₂-C₆-Alkenyl, C₃-C₆-Cycloalkyl und C₅-C₆-Cycloalkenyl ausgewählt ist,
n für 0 oder 1 steht und
die gestrichelte Linie zusammen mit der C-C-Bindung eine Einfachbindung oder eine Doppelbindung darstellt.

4. Verbindung nach Anspruch 1, ausgewählt aus der Gruppe bestehend aus 2-(But-2-en-2-yl)-4-ethyl-2,3,4,6,7,8-hexahydro-5H-chromen-5-on; 2-(But-2-en-2-yl)-4-methyl-2,3,4,6,7,8-hexahydro-5H-chromen-5-on; 2-(But-3-en-2-yl)-4-ethyl-2,3,4,6,7,8-hexahydro-5H-chromen-5-on; 2-(But-3-en-2-yl)-4-methyl-2,3,4,6,7,8-hexahydro-5H-chromen-5-on; 2-(sec-Butyl)-4-ethyl-2,3,4,6,7,8-hexahydro-5H-chromen-5-on; 2-(sec-Butyl)-4-methyl-2,3,4,6,7,8-hexahydro-5H-chromen-5-on; 2-(tert-Butyl)-4-ethyl-2,3,4,6,7,8-hexahydro-5H-chromen-5-on; 2,2,4-Trimethyl-2,3,4,6,7,8-hexahydro-5H-chromen-5-on; 2,3,4-Trimethyl-4,6,7,8-tetrahydro-5H-chromen-5-on; 2,4-Diethyl-2,3,4,6,7,8-hexahydro-5H-chromen-5-on; 2,4-Diethyl-3,4,6,7-tetrahydrocyclopenta[b]pyran-5(2H)-on; 2,4-Diethyl-3-methyl-2,3,4,6,7,8-hexahydro-5H-chromen-5-on; 2,4-Diethyl-4,6,7,8-tetrahydro-5H-chromen-5-on; 2,4-Dimethyl-2,3,4,6,7,8-hexahydro-5H-chromen-5-on; 2,4-Dimethyl-2,3,4,6,7,8-hexahydro-5H-chromen-5-on; 2,4-Dimethyl-4,6,7,8-tetrahydro-5H-chromen-5-on; 2-Allyl-4-ethyl-2,3,4,6,7,8-hexahydro-5H-chromen-5-on; 2-Allyl-4-methyl-2,3,4,6,7,8-hexahydro-5H-chromen-5-on; 2-Butyl-4-ethyl-2,3,4,6,7,8-hexahydro-5H-chromen-5-on; 2-Butyl-4-methyl-2,3,4,6,7,8-hexahydro-5H-chromen-5-on; 2-Cyclopropyl-4-ethyl-2,3,4,6,7,8-hexahydro-5H-chromen-5-on; 2-Cyclopropyl-4-methyl-2,3,4,6,7,8-hexahydro-5H-chromen-5-on; 2-Ethyl-3,4-dimethyl-2,3,4,6,7,8-hexahydro-5H-chromen-5-on; 2-Ethyl-4-(prop-1-en-1-yl)-2,3,4,6,7,8-hexahydro-5H-chromen-5-on; 2-Ethyl-4,7-dimethyl-2,3,4,6,7,8-hexahydro-5H-chromen-5-on; 2-Ethyl-4-methyl-2,3,4,6,7,8-hexahydro-5H-chromen-5-on; 2-Ethyl-4-propyl-2,3,4,6,7,8-hexahydro-5H-chromen-5-on; 2-Isobutyl-4-methyl-2,3,4,6,7,8-hexahydro-5H-chromen-5-on; 2-Isopropyl-4-methyl-2,3,4,6,7,8-hexahydro-5H-chromen-5-on; 2-Isopropyl-4-methyl-2,3,4,6,7,8-hexahydro-5H-chromen-5-on; 4-Ethyl-2-(2-methylallyl)-2,3,4,6,7,8-hexahydro-5H-chromen-5-on; 4-Ethyl-2-(2-methylprop-1-en-1-yl)-2,3,4,6,7,8-hexahydro-5H-chromen-5-on; 4-Ethyl-2-(prop-1-en-1-yl)-2,3,4,6,7,8-hexahydro-5H-chromen-5-on; 4-Ethyl-2-(prop-1-en-2-yl)-2,3,4,6,7,8-hexahydro-5H-chromen-5-on; 4-Ethyl-2,3-dimethyl-2,3,4,6,7,8-hexahydro-5H-chromen-5-on; 4-Ethyl-2-isobutyl-2,3,4,6,7,8-hexahydro-5H-chromen-5-on; 4-Ethyl-2-isopropyl-2,3,4,6,7,8-hexahydro-5H-chromen-5-on; 4-Ethyl-2-methyl-2,3,4,6,7,8-hexahydro-5H-chromen-5-on; 4-Ethyl-2-propyl-2,3,4,6,7,8-hexahydro-5H-chromen-5-on; 4-Ethyl-2-propyl-3,4,6,7-tetrahydrocyclopenta[b]pyran-5(2H)-on; 4-Ethyl-2-vinyl-2,3,4,6,7,8-hexahydro-5H-chromen-5-on; 4-Isopropyl-2-methyl-2,3,4,6,7,8-hexahydro-5H-chromen-5-on; 4-Methyl-2-(2-methylprop-1-en-1-yl)-2,3,4,6,7,8-hexahydro-5H-chromen-5-on; 4-Methyl-2-(prop-1-en-2-yl)-2,3,4,6,7,8-hexahydro-5H-chromen-5-on und 4-Methyl-2-propyl-2,3,4,6,7,8-hexahydro-5H-chromen-5-on und Mischungen davon.

5. Duftstoffzusammensetzung, umfassend eine Verbindung der Formel (I) gemäß einem der Ansprüche 1 bis 4 oder eine Mischung davon.

6. Duftstoffhaltiger Artikel, umfassend eine Verbindung der Formel (I) gemäß einem der Ansprüche 1 bis 4 oder eine Mischung davon und eine Konsumproduktbasis.

7. Duftstoffhaltiger Artikel nach Anspruch 6, wobei die Konsumproduktbasis aus Feinparfümerie, Körperpflegeprodukten und Textilpflegeprodukten ausgewählt ist.

8. Verwendung einer Verbindung der Formel (I) gemäß einem der Ansprüche 1 bis 4 oder einer Mischung davon als Duftstoff.

9. Verfahren zur Verbesserung, Verstärkung und/oder Modifizierung einer Konsumproduktbasis durch Zugabe einer olfaktorisch annehmbaren Menge einer Verbindung der Formel (I) gemäß einem der Ansprüche 1 bis 4 oder einer Mischung davon.

## Revendications

1. Composé de formule (I) 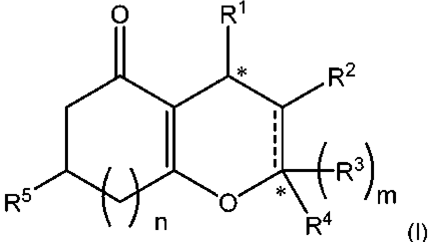 dans laquelle
R¹ est choisi parmi C₁ - C₅ alkyle,
R² est choisi parmi hydrogène, C₁-C₃ alkyle,
R³ est choisi parmi hydrogène, méthyle et éthyle,
R⁴ est choisi parmi C₁ - C₆ alkyle, C₂-C₆ alcényle, C₃-C₆ cycloalkyle, et C₅-C₆ cycloalcényle,
R⁵ est choisi parmi hydrogène et méthyle,
n est 0 ou 1, et
la ligne pointillée représente conjointement avec la liaison C-C une simple liaison et m est 1, ou
la ligne pointillée représente conjointement avec la liaison C-C une double liaison et m est 0.

2. Composé selon la revendication 1, dans lequel le composé de formule (I) est un composé de formule (II) 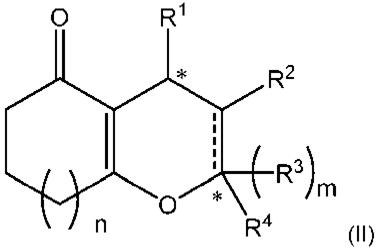 dans laquelle
R¹ est choisi parmi C₁ - C₅ alkyle,
R² est choisi parmi hydrogène, C₁-C₃ alkyle,
R³ est choisi parmi hydrogène, méthyle et éthyle,
R⁴ est choisi parmi C₁ - C₆ alkyle, C₂-C₆ alcényle, C₃-C₆ cycloalkyle, et C₅-C₆ cycloalcényle,
n est 0 ou 1, et
la ligne pointillée représente conjointement avec la liaison C-C une simple liaison et m est 1, ou
la ligne pointillée représente conjointement avec la liaison C-C une double liaison et m est 0.

3. Composé selon la revendication 1, dans lequel le composé de formule (I) est un composé de formule (III) 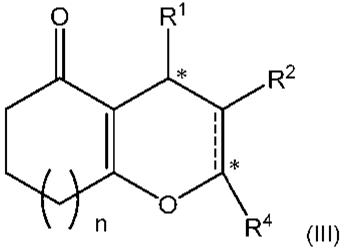 dans laquelle
R¹ est choisi parmi C₁ - C₅ alkyle,
R² est choisi parmi hydrogène, C₁-C₃ alkyle,
R⁴ est choisi parmi C₁ - C₆ alkyle, C₂-C₆ alcényle, C₃-C₆ cycloalkyle, et C₅-C₆ cycloalcényle,
n est 0 ou 1, et
la ligne pointillée représente conjointement avec la liaison C-C une simple liaison ou une double liaison.

4. Composé selon la revendication 1 choisi dans le groupe constitué par 2-(but-2-én-2-yl)-4-éthyl-2,3,4,6,7,8-hexahydro-5H-chromén-5-one ; 2-(but-2-én-2-yl)-4-méthyl-2,3,4,6,7,8-hexahydro-5H-chromén-5-one ; 2-(but-3-én-2-yl)-4-éthyl-2,3,4,6,7,8-hexahydro-5H-chromén-5-one ; 2-(but-3-én-2-yl)-4-méthyl-2,3,4,6,7,8-hexahydro-5H-chromén-5-one ; 2-(sec-butyl)-4-éthyl-2,3,4,6,7,8-hexahydro-5H-chromén-5-one ; 2-(sec-butyl)-4-méthyl-2,3,4,6,7,8-hexahydro-5H-chromén-5-one ; 2-(tert-butyl)-4-éthyl-2,3,4,6,7,8-hexahydro-5H-chromén-5-one ; 2,2,4-triméthyl-2,3,4,6,7,8-hexahydro-5H-chromén-5-one ; 2,3,4-triméthyl-4,6,7,8-tétrahydro-5H-chromén-5-one ; 2,4-diéthyl-2,3,4,6,7,8-hexahydro-5H-chromén-5-one ; 2,4-diéthyl-3,4,6,7-tétrahydrocyclopenta[b]pyran-5(2H)-one ; 2,4-diéthyl-3-méthyl-2,3,4,6,7,8-hexahydro-5H-chromén-5-one ; 2,4-diéthyl-4,6,7,8-tétrahydro-5H-chromén-5-one ; 2,4-diméthyl-2,3,4,6,7,8-hexahydro-5H-chromén-5-one ; 2,4-diméthyl-2,3,4,6,7,8-hexahydro-5H-chromén-5-one ; 2,4-diméthyl-4,6,7,8-tétrahydro-5H-chromén-5-one ; 2-allyl-4-éthyl-2,3,4,6,7,8-hexahydro-5H-chromén-5-one ; 2-allyl-4-méthyl-2,3,4,6,7,8-hexahydro-5H-chromén-5-one ; 2-butyl-4-éthyl-2,3,4,6,7,8-hexahydro-5H-chromén-5-one ; 2-butyl-4-méthyl-2,3,4,6,7,8-hexahydro-5H-chromén-5-one ; 2-cyclopropyl-4-éthyl-2,3,4,6,7,8-hexahydro-5H-chromén-5-one ; 2-cyclopropyl-4-méthyl-2,3,4,6,7,8-hexahydro-5H-chromén-5-one ; 2-éthyl-3,4-diméthyl-2,3,4,6,7,8-hexahydro-5H-chromén-5-one ; 2-éthyl-4-(prop-1-én-1-yl)-2,3,4,6,7,8-hexahydro-5H-chromén-5-one ; 2-éthyl-4,7-diméthyl-2,3,4,6,7,8-hexahydro-5H-chromén-5-one ; 2-éthyl-4-méthyl-2,3,4,6,7,8-hexahydro-5H-chromén-5-one ; 2-éthyl-4-propyl-2,3,4,6,7,8-hexahydro-5H-chromén-5-one ; 2-isobutyl-4-méthyl-2,3,4,6,7,8-hexahydro-5H-chromén-5-one ; 2-isopropyl-4-méthyl-2,3,4,6,7,8-hexahydro-5H-chromén-5-one ; 2-isopropyl-4-méthyl-2,3,4,6,7,8-hexahydro-5H-chromén-5-one; 4-éthyl-2-(2-méthylallyl)-2,3,4,6,7,8-hexahydro-5H-chromén-5-one; 4-éthyl-2-(2-méthylprop-1-én-1-yl)-2,3,4,6,7,8-hexahydro-5H-chromén-5-one; 4-éthyl-2-(prop-1-én-1-yl)-2,3,4,6,7,8-hexahydro-5H-chromén-5-one; 4-éthyl-2-(prop-1-én-2-yl)-2,3,4,6,7,8-hexahydro-5H-chromén-5-one; 4-éthyl-2,3-diméthyl-2,3,4,6,7,8-hexahydro-5H-chromén-5-one; 4-éthyl-2-isobutyl-2,3,4,6,7,8-hexahydro-5H-chromén-5-one; 4-éthyl-2-isopropyl-2,3,4,6,7,8-hexahydro-5H-chromén-5-one; 4-éthyl-2-méthyl-2,3,4,6,7,8-hexahydro-5H-chromén-5-one ; 4-éthyl-2-propyl-2,3,4,6,7,8-hexahydro-5H-chromén-5-one ; 4-éthyl-2-propyl-3,4,6,7-tétrahydrocyclopenta[b]pyran-5(2H)-one ; 4-éthyl-2-vinyl-2,3,4,6,7,8-hexahydro-5H-chromén-5-one ; 4-isopropyl-2-méthyl-2,3,4,6,7,8-hexahydro-5H-chromén-5-one ; 4-méthyl-2-(2-méthylprop-1-én-1-yl)-2,3,4,6,7,8-hexahydro-5H-chromén-5-one ; 4-méthyl-2-(prop-1-én-2-yl)-2,3,4,6,7,8-hexahydro-5H-chromén-5-one; et 4-méthyl-2-propyl-2,3,4,6,7,8-hexahydro-5H-chromén-5-one; et leurs mélanges.

5. Composition de fragrance comprenant un composé de formule (I) tel que défini dans l'une quelconque des revendications 1 à 4, ou un mélange de ceux-ci.

6. Article fragrancé comprenant un composé de formule (I) tel que défini dans l'une quelconque des revendications 1 à 4, ou un mélange correspondant, et une base de produit de consommation.

7. Article fragrancé selon la revendication 6, dans lequel la base de produit de consommation est choisie parmi une parfumerie fine, des produits de soins personnels et des produits d'entretien de tissu.

8. Utilisation comme fragrance d'un composé de formule (I) tel que défini dans l'une quelconque des revendications 1 à 4, ou d'un mélange correspondant.

9. Procédé d'amélioration, d'augmentation et/ou de modification d'une base de produit de consommation au moyen d'un ajout à celle-ci d'une quantité acceptable sur le plan olfactif d'un composé de formule (I) tel que défini dans l'une quelconque des revendications 1 à 4, ou d'un mélange correspondant.
